# EUROPEAN PATENT SPECIFICATION

(11) **EP 0 638 552 B1**
(45) Date of publication and mention of the grant of the patent: **04.06.2003**
(21) Application number: 94906364.8
(22) Date of filing: 10.02.1994
(51) Int. Cl.: C07D 205/08

(54) **PROCESS FOR SYNTHESIZING 4-SUBSTITUTED AZETIDINONE DERIVATIVE**
VERFAHREN ZUR SYNTHESE VON 4-SUBSTITUIERTEN AZETIDINON-DERIVATEN
PROCEDE D'OBTENTION PAR SYNTHESE D'UN DERIVE D'AZETIDINONE EN SUBSTITUTION 4

(30) Priority: 12.02.1993 JP 4755293
(43) Date of publication of application: 15.02.1995
(73) Proprietor: Daiichi Suntory Pharma Co., Ltd., Tokyo (JP)
(72) Inventor: ISHIGURO, Masaji, Hyogo 665 (JP); NAKATSUKA, Takashi, Shimamoto-cho, Mishima-gun Osaka 618 (JP); TANAKA, Rie, Ibaraki-shi Osaka 567 (JP); SHIMAMOTO, Tetsuo, Osaka 565 (JP); YOSHIDA, Takuro, Gunma 374 (JP)
(74) Representative: Burford, Anthony Frederick
(86) International application number: JP9400195
(87) International publication number: WO94018163

(56) References cited:
- EP-A- 0 045 198
- EP-A- 0 546 742
- JP-A- 56 150 058
- JP-A- 56 154 458
- JP-A- 59 046 265
- JP-A- 61 165 365
- JP-A- 62 246 550

## Description

The present invention relates to a process for producing 4-substituted azetidinone derivatives which are important as synthetic intermediates, for example, for carbapenem based antimicrobial agents.

There have already been reported several useful processes (for example, JP-A-61-207373) for producing azetidinone derivatives represented by the general formula [1] : 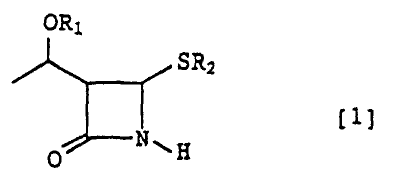 (wherein OR₁ is a protected hydroxyl group; R₂ is a substituted or unsubstituted alkyl group, substituted or unsubstituted alkenyl group or substituted or unsubstituted aromatic group).
To produce from such azetidinone derivatives of the general formula [1] 4-substituted azetidinone derivatives represented by the general formula [3]: 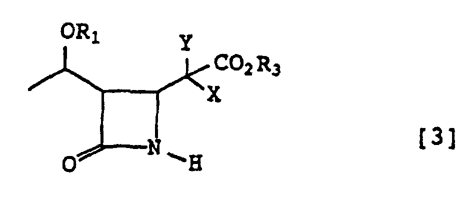 (wherein OR₁ is as defined above; CO₂R₃ is an esterified carboxyl group; X and Y are the same or different and represent individually a substituted or unsubstituted alkyl group, substituted or unsubstituted alkenyl group, substituted or unsubstituted aralkyl group, substituted or unsubstituted aryl group, substituted or unsubstituted . alkylthio group, substituted or unsubstituted alkenylthio group, substituted or unsubstituted aralkylthio group, substituted or unsubstituted arylthio group, substituted or unsubstituted alkyloxy group, substituted or unsubstituted alkenyloxy group, substituted or unsubstituted aralkyloxy group, substituted or unsubstituted aryloxy group, substituted or unsubstituted silyloxy group, substituted or unsubstituted heterocyclic group, substituted or unsubstituted heterocyclic-thio group, substituted or unsubstituted heterocyclic-oxy group, substituted or unsubstituted acyl group, substituted or unsubstituted ester group, substituted or unsubstituted thioester group, substituted or unsubstituted amide group, substituted or unsubstituted amino group, hydrogen atom or halogen atom, or cooperate with each other to form a substituted or unsubstituted cycloalkane-2-on-1-yl group),
it has been necessary to oxidize the sulfide group of the azetidinone derivative of the general formula [1] to thereby convert the group to the more readily removable sulfone group or substitute an acyloxy group for the sulfide group.

In order to convert the azetidinone derivative of the general formula [1] to the corresponding sulfone-containing derivative, there is known a method utilizing peroxyacid (Yoshida et al., Chem. Pharm. Bull., 29, 2899 (1981)), while a method using a mercury compound (Yoshida et al., Chem. Pharm. Bull., 29, 2899 (1981)) is known for the conversion of the same to the acyloxy-containing derivative, but difficulties are encountered in bringing these methods into commercial practice in terms of inter alia hygiene, safety or toxicity.

Referring to the conversion of [1] to the acyloxy derivative, there has recently been reported a useful method involving the use of a copper compound (JP-A-3-163057), resulting in marked improvement in the commercial production of the said derivative. Nevertheless, this method requires heating in carrying out the reaction and is yet to be improved. On the other hand, there have been reported a method (JP-A-3-157365) of directly converting the azetidinone derivative of the general formula [1] to the azetidinone derivative of the general formula [3]: 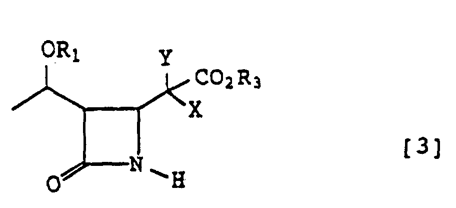 (wherein OR₁, CO₂R₃, X and Y are as defined above) and also a method (Ito et al., Tetrahedron, 47, 2801 (1991)) of converting an acyloxyazetidinone derivative to a azetidinone derivative of the general formula [3], which methods have been developed with a specific view to introducing a substituent into the 1-methylene group to enhance the chemical and in vivo stabilities of carbapenem based antimicrobial compounds. However, these methods do not allow the ester compound to be utilized directly as a substitution reagent; before the said reactions are carried out, the former requires the ester compound to be converted to the corresponding diazonium reagent, while the latter necessitates the conversion of the same to the corresponding oxazolidone reagent.

A method of converting the azetidinone derivative of the general formula [1] directly to the azetidinone derivative of the general formula [3] constitutes a process having one step less as compared with the method of converting the azetidinone derivative of [1] to the sulfone-containing or acyloxy-containing derivative to thereby effect the intended synthesis, and could consequently offer a useful, advantageous synthetic means, only if the method can be brought to practice by a simple and practical operation. If the method permits the reaction to be carried out at lower temperatures and can furthermore allow a readily available reagent to be used in the introduction of a substituent at the 1-methylene group directly without being converted to another reagent, in addition, substantial improvement could be achieved. In view of the above, the present inventors conducted investigations into a method permitting the azetidinone derivative of the general formula [1] to be converted directly to the azetidinone derivative of the general formula [3] under mild conditions, and have found the present invention.

US-A-4287123 discloses the preparation of the carbapenem antibiotic thienamycin from a 3-acyloxy-4-hydrocarbyloxycarbonylmethyl-2-azetidinone.

EP-A-0102239 relates to 1-phosphoranylidenemethyl-4-(hydrocarbyl- or heterocyclic- thio)carbonylmethyl-2-azetidinone intermediates for the preparation of carbapenem derivatives. They are prepared by reaction of the corresponding 1-(protected) carboxycarbonyl derivative by reaction with a triorganophosphorus compound.

US-A-5075435 discloses certain 4-methylethenyl-2-azetidinone derivatives as intermediates in the preparation of carbapenem derivatives.

US-A-5075437 discloses the preparation of 4-(1-alkyl-2-oxo-ethyl)-2-azetidinone derivatives substituted at the 2-position of the 4-substituted ethyl group by 1-alkyl-1-(protected)hydroxy-alkyl or 2-thioxothiazolidin-3-yl by reacting a 4-acyloxy-2-azetidinone derivative with a corresponding ketone in the presence of an enolizating agent selected from stannous alkylsulfonate and stannous perhaloalkylsulfonate.

EP-A-0045198 discloses the preparation of carbapenem compounds by cyclization of 1-(1-phosphonio or phosphono) activated carboxymethyl-4-hydrocarbyl (or hydrocarbylthio) carbonylmethyl 2-azetidinones.

EP-A-0546742 relates to 4-(1,1-dialkoxycarbonylalkyl) azetidin-2-one intermediates for the preparation of carbapenem antibiotics. In particular, they are deesterified and decarboxylated to form 4-(1-carboxyalkyl)-azetidin-2-one derivatives (see Formula II).

The present inventors found that an ester compound represented by the general formula [2] : 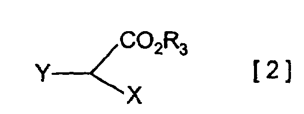 (wherein CO₂R₃, X and Y are as defined above) can be treated with a metal base to give the corresponding metal enolate, which can then be reacted with an azetidinone derivative of the general formula [1]: 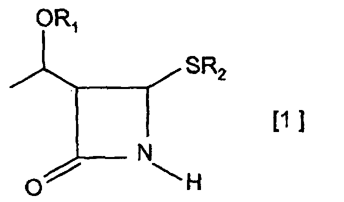 (wherein OR₁ and R₂ are as defined above) in the presence of a copper compound to produce a 4-4-substituted azetidinone derivative of the general formula [3]: 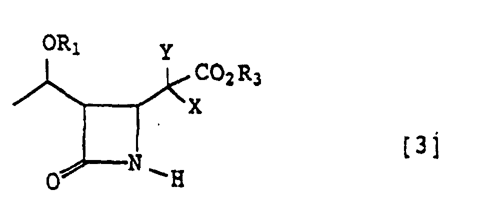 (wherein OR₁, CO₂R₃, X and Y are as defined above).

It was found also that an ester compound of the general formula [2]: 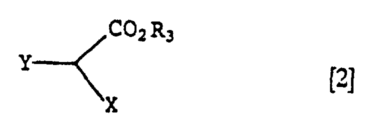 (wherein CO₂R₃, X and Y are as defined above) can be reacted with an azetidinone derivative of the general formula [1]: 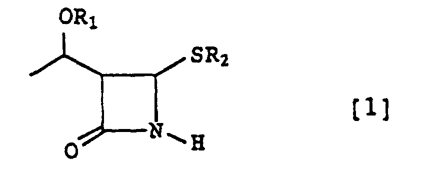 (wherein OR₁ and R₂ are as defined above) in the presence of zinc and copper compounds to produce a 4-substituted azetidinone derivative of the general formula [3]: 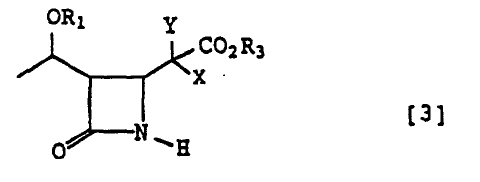 (wherein OR₁, CO₂R₃, X and Y are as defined above).

With reference to the decarboxylation of 4-substituted azetidinone derivatives represented by the general formula [4]: 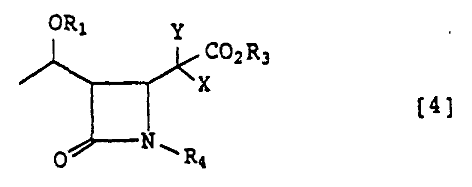 (wherein OR₁, CO₂R₃, X and Y are as defined above and R₄ is a hydrogen atom or a protective group or substituent group for amino group),
there has been reported a method (JP-A-5-155850) involving the selection of substituents for R₄ to thereby control the configurations of X and Y, and by virtue of the method, highly industrially useful 1-B- substituted derivatives can be produced preferentially. Consequently, the 4-substituted azetidinone derivatives of the general formula [3] as synthesized by the reaction according to the present invention and the 4-substituted azetidinone derivatives of the general formula [4] wherein R₄ is a protective group or a substituent group for amino group readily derived from those by conventional methods, and the 4-substituted azetidinone derivatives of the general formula [5]: 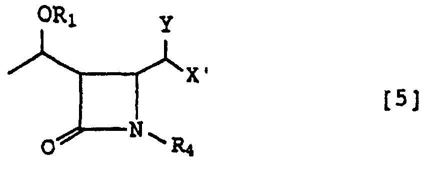 (wherein OR₁, R₄ and Y are as defined above; X' is the same as defined for X or a mercapto, hydroxyl, formyl, carboxyl or thiocarboxyl group),
which are obtained by converting such derivative to a carboxylic acid compound, followed by decarboxylation, can be utilized as starting compounds for carbapenem compounds of utility as therapeutic agents for infections.

Such being the case, it was also found that the 4-substituted azetidinone derivatives of the general formulae [3] and [4] can be converted to their carboxylic acid compounds, for example by way of hydrolysis or a reaction procedure selected for the type of ester, followed by decarboxylation treatment to give 4-substituted azetidinone derivatives of the general formula [5] useful as starting materials for carbapenem compounds.

The present invention is based on such novel findings and relates to (A) a process for synthesizing 4-substituted azetidinone derivatives of the above-described general formula [3], characterized in that said process comprises reacting an azetidinone derivative of the general formula [1] with an ester compound of the above-described general formula [2] in the presence of zinc and copper compounds; (B) a process for synthesizing 4-substituted azetidinone derivatives of the above-described general formula [3], characterized in that said process comprises treating an ester compound of the above-described general formula [2] with a metal base to give a metal enolate, which is then reacted with an azetidinone derivative of the above-mentioned general formula [1] in the presence of a copper compound; (C) a process for synthesizing 4-substituted azetidinone derivatives of the above-mentioned general formula [5], characterized in that said process comprises converting an ester compound of the above-mentioned general formula [3] or [4] to a carboxylic acid compound, followed by decarboxylation treatment; and (D) novel 4-substituted azetidinone derivatives among the compounds represented by the above-mentioned general formulae [3], [4] and [5] in the above-mentioned processes.

The substitution reaction for the 4-position of the azetidinone derivative of the general formula [1] is normally carried out in hydrocarbon solvents such as benzene and toluene, chlorinated hydrocarbon solvents such as methylene chloride and chloroform, nitrile solvents such as acetonitrile, ketone solvents such as acetone and methyl vinyl ketone, ether solvents such as diethyl ether and tetrahydrofuran, ester solvents such as ethyl acetate, or mixtures thereof.

The ester compound of the general formula [2] is desirably used in proportions of 1 to 3 equivalents against the azetidinone derivative of the general formula [1]. The ester compound is preferably acted on by an equal amount of a metal base to produce the metal enolate, followed by addition of the azetidinone derivative of the general formula [1] and a copper compound to allow the reaction. Alternatively, when the ester compound of the general formula [2] is reacted with the azetidinone derivative of the general formula [1] directly without converting to the corresponding metal enolate, the reaction is effected in the above-mentioned solvents in the presence of zinc and copper compounds, preferably using the ester compound of the general formula [2] in proportions of 1 to 3 equivalents against the azetidinone derivative of the general formula [1].

As the copper compound, there may be mentioned, for example, copper oxides, copper halides, salts of copper with organic carboxylic acids, salts of copper with mineral acids, and their complexes. The preferred examples include cuprous oxide, cupric oxide, cuprous chloride, cupric chloride, cuprous bromide, cupric fluoride, cuprous iodide, cupric perchlorate, cupric nitrate, cupric sulfate, cuprous sulfide, cupric sulfide, cupric trifluoromethanesulfonate, copper cyanide, salts of copper with aliphatic carboxylic acids such as cuprous acetate, cupric acetate, cupric trifluoroacetate, copper propionate and copper butyrate, and salts of copper with aromatic carboxylic acids such as copper benzoate There are usually employed cuprous halides and their complexes, with cuprous bromide dimethyl sulfide complex being particularly preferred. The used amount each of the copper and zinc compounds is suitably in the range of 1 to 4 equivalents against the azetidinone derivative of the general formula [1]. Meanwhile, preferred examples of the metal base include alkali metal hydride compounds such as sodium hydride and potassium hydride. The reaction temperature varies depending upon the type of the used ester compound of the general formula [2] and the kind of the azetidinone derivative of the general formula [1] and is usually in the range of 0°C to 50°C, suitably in the region of 0°C to room temperature.

The conversion of the 4-substituted azetidinone derivative of the general formula [3] to the 4-substituted azetidinone derivative of the general formula [4] where R₄ is a protective group or substituent for the amino group can be done by conventional procedures.

Referring to the post-reaction treatment, a saturated ammonium chloride solution can be added to the reaction solution, followed by extraction of the solution mixture with an organic solvent, and the organic extract layer washed with water, dried and concentrated to give the objective compound. The crude reaction product can be used in the subsequent reaction directly without being purified, but can also be purified by recrystallization or column chromatography, if necessary.

The conversion reaction of the ester compound of the general formulae [3] and [4] to the corresponding carboxylic acid compound is usually carried out in pyridine based solvents such as pyridine, lutidine and collidine, nitrile based solvents such as acetonitrile, ketone based solvents such as acetone and methyl vinyl ketone, ether based solvents such as tetrahydrofuran, alcohol based solvents such as methanol and ethanol, or mixtures thereof, suitably in pyridine, collidine or tetrahydrofuran, by hydrolysis in the presence of a base, for example, by adding an aqueous solution of a metal hydroxide compound to the compound of the general formulae [3] and [4] or under conditions of a selective procedure adopted according to the type of the ester compound.

The conditions of the selective reaction procedure according to the type of the ester compound can be exemplified by reaction utilizing metals such as palladium for allyl esters, catalytic hydrogenation procedures for benzyl esters and procedures utilizing zinc for trichloroethyl esters and these all are known and can be carried out in accordance with descriptions given in the literature.

The metal hydroxide compound is preferably used in proportions of 1 to 2 equivalents against the ester compound to conduct the reaction at a temperature in the range of normally 0°C to 50°C, preferably 0°C to room temperature. Examples of the metal hydroxide compound include alkali metal hydroxide compounds such as sodium hydroxide, potassium hydroxide and lithium hydroxide, and alkaline earth metal hydroxide compounds such as barium hydroxide.

The decarboxylation of the carboxylic acid compound to form the 4-substituted azetidinone derivative of the general formula [5] can be carried out, for example, by adding an acid in an amount equivalent to the metal hydroxide compound used in the hydrolysis to give the carboxylic acid compound, followed by heating normally at 50°C to 200°C, preferably 100°C to 150°C. The acid can be exemplified by mineral acids such as hydrochloric acid, sulfuric acid and nitric acid, organic acids such as acetic acid, propionic acid and camphorsulfonic acid, and ion exchange resins.

The post-reaction treatment can be effected by concentrating the reaction solution, extracting the concentrate with organic solvent and washing the organic extract layer with water, followed by drying and concentrating to produce the objective compound. The crude reaction product can be used in the subsequent reaction directly without being purified, but can also be purified by recrystallization or column chromatography, if necessary.

The removal procedure of the protective group for the amino group in R₄ of the 4-substituted azetidinone derivative of the general formula [5] varies with the type of the protective group, and can be conducted by selecting an appropriate reaction; when the protective group is a tri-substituted silyl group, for example, a weak acid such as dilute hydrochloric acid may be reacted with the azetidinone derivative of [5], while in the case of the protective group being benzyl, phenethyl or benzhydryl group which may be substituted, metallic sodium may be reacted with the derivative of [5] in liquid ammonia by the Barch's reduction.

The 4-substituted azetidinone derivative of the general formula [5] as produced according to the present invention can be utilized, directly or after removal or replacement of the amino protective group, as a starting compound for carbapenem based compounds.

The conversion procedure to a carbapenem based compound varies depending upon the kind of substituent at the 4-position, and can be conducted by a suitably selected reaction, for example, in accordance with the descriptions on the cyclization reaction (Hatanaka, M. et al., Tetrahedron Lett., 1981, 22, 3883) by way of Diechmann reaction, reductive cyclization reaction (Shibata, T. et al., J. Antibiotics, 1989, 42, 374) with use of phosphite, cyclization reaction (Guthikonda, R. N. et al., J. Med. Chem., 1987, 30, 871) based on Wittig reaction, or cyclization reaction with use of transitional metals (Ratcliffe, R. W. et al., Tetrahedron Lett., 1980, 21, 1193).

The protected hydroxyl group represented by OR1 is not particularly limited and comprehends hydroxyl groups protected with protective groups conventionally used for the hydroxyl group, which are exemplified by tri-substituted silyloxy groups, specifically trialkylsilyloxy, aryl(alkyl)alkoxysilyloxy, alkoxydiarylsilyloxy, triarylsilyloxy, alkyldiarylsilyloxy, aryldialkylsilyloxy and triaralkylsilyloxy groups, such as trimethylsilyloxy, triethylsilyloxy, triisopropylsilyloxy, dimethylhexylsilyloxy, tert-butyldimethylsilyloxy, methyldiisopropylsilyloxy, isopropyldimethylsilyloxy, tert-butylmethoxyphenylsilyloxy, tert-butoxydiphenylsilyloxy, triphenylsilyloxy, tert-butyldiphenylsilyloxy, dimethylcumylsilyloxy and tribenzylsilyloxy groups; lower (as hereinafter defined) alkoxy groups which may have at least one suitable substituent, such as methoxymethoxy, methoxyethoxymethoxy and triphenylmethoxy groups; lower alkanoyloxy groups which may have at least one suitable substituent, such as acetoxy, chloroacetoxy, methoxyacetoxy, propionyloxy, butyryloxy, isobutyryloxy, valeryloxy, pivaloyloxy, hexanoyloxy, 2-ethylbutyryloxy, 3,3-dimethylbutyryloxy and pentanoyloxy groups; lower alkoxycarbonyloxy groups which may have at least one suitable substituent, such as methoxycarbonyloxyl ethoxycarbonyloxy, propoxycarbonyloxy, isopropoxycarbonyloxy, tert-butoxycarbonyloxy, 2-iodoethoxycarbonyloxy, 2, 2-dichloroethoxycarbonyloxy and 2,2,2-trichloroethoxycarbonyloxy groups; lower alkenyloxycarbonyloxy groups which may have at least one suitable substituent, such as vinyloxycarbonyloxy, allyloxycarbonyloxy and 2-chloroallyloxycarbonyloxy groups; arylcarbonyloxy groups which may have at least one suitable substituent, such as benzoyloxy group; aralkyloxycarbonyloxy groups which may have at least one suitable substituent, such as benzyloxycarbonyloxy, p-nitrobenzyloxycarbonyloxy, p-methoxybenzyloxycarbonyloxy, phenethyloxycarbonyloxy, trityloxycarbonyloxy, benzhydryloxycarbonyloxy, bis-(methoxyphenyl)methyloxycarbonyloxy, 3,4-dimethoxybenzyloxycarbonyloxy and 4-hydroxy-3,5-di-tert-butylbenzyloxycarbonyloxy groups; aryloxycarbonyloxy groups which may have at least one substituent, such as phenyloxycarbonyloxy, 4-chlorophenyloxycarbonyloxy, tolyloxycarbonyloxy, tert-butylphenyloxycarbonyloxy, xylyloxycarbonyloxy, mesityloxycarbonyloxy and cumenyloxycarbonyloxy groups; aralkyloxy groups which may have at least one substituent, such as benzyloxy, p-nitrobenzyloxy, p-methoxybenzyloxy, p-tert-butylbenzyloxy, 3,4-dimethylbenzyloxy, 2,4-dimethoxybenzyloxy, benzhydryloxy and trityloxy groups; heterocyclic-oxy groups which may have at least one . suitable substituent, such as tetrahydropyranyloxy group. Unless otherwise specified particularly, the term "lower" designates the same meaning in the below description throughout the present specification, and means preferably 1 to 6 carbon atoms, particularly preferably 1 to 4 carbon atoms.

The group represented by R₂ is not particularly limited, but examples of such group, which are readily available and can be produced at low costs, include alkyl groups being exemplified by linear or branched lower alkyl groups such as methyl, ethyl, n-propyl, isopropyl, n-butyl, tert-butyl and hexyl groups and monocyclic or polycyclic alkyl groups such as cyclopentyl, cyclohexyl, menthyl, fenchyl and bornyl groups; alkenyl groups being exemplified by linear or branched alkenyl groups such as vinyl, allyl, 1-propenyl, 2-butenyl and 2-methyl-2-propenyl groups; aromatic groups being exemplified by aryl groups having 6 to 10 carbon atoms such as phenyl, tolyl, xylyl, mesityl and cumenyl groups and aromatic heterocyclic groups such as pyridyl, pyrimidinyl, pyrazinyl and pyridazinyl groups, wherein such alkyl, alkenyl, and aromatic groups individually may be substituted with one or not less than two substituents, for example, halogen atoms such as fluorine, chlorine and bromine atoms; linear or branched lower alkyl groups such as methyl, ethyl, n-propyl, isopropyl, n-butyl, tert-butyl and hexyl groups; monocyclic or polycyclic alkyl groups such as cyclopentyl, cyclohexyl, menthyl, fenchyl and bornyl groups; linear or branched lower alkoxy groups such as methoxy and ethoxy groups; carboxyl group; amino group; nitro group; cyano group; hydroxyl group; aryl groups having 6 to 10 carbon atoms, such as phenyl, tolyl, xylyl, mesityl and cumenyl groups, which may be substituted with the above-mentioned halogen atoms, and lower alkyl, lower alkoxy, carboxyl, amino, nitro, cyano and hydroxyl groups; aralkyl groups having 7 to 24 carbon atoms, such as benzyl, phenethyl, trityl and benzhydryl groups, which may be substituted with the above-mentioned halogen atoms, and lower alkyl, lower alkoxy, carboxyl, amino, nitro, cyano and hydroxyl groups.

The group represented by R₃ is not particularly limited, only if it can eliminate from the esterified carboxyl group represented by CO₂R₃ through hydrolysis or under conditions of the selective procedure according to the type of ester, and its preferred examples include those capable of forming the following esters:
Tri-substituted silyl esters such as trialkylsilyl esters, aryl(alkyl)alkoxysilyl esters, alkoxydiarylsilyl esters, triarylsilyl esters, alkyldiarylsilyl esters, aryldialkylsilyl esters, triaralkylsilyl esters (e.g. trimethylsilyl, triethylsilyl, triisopropylsilyl, dimethylhexylsilyl, tertbutyldimethylsilyl, methyldiisopropylsilyl, isopropyldimethylsilyl, tert-butylmethoxyphenylsilyl, tert-butoxydiphenylsilyl, triphenylsilyl, tert-butyldiphenylsilyl, dimethylcumylsilyl and tribenzylsilyl esters); tri-substituted silyl lower-alkyl esters such as trialkylsilyl lower-alkyl esters, aryl(alkyl)alkoxysilyl lower-alkyl esters, alkoxydiarylsilyl lower-alkyl esters, triarylsilyl lower-alkyl esters, alkyldiarylsilyl lower-alkyl esters, aryldialkylsilyl lower-alkyl esters, and triaralkylsilyl lower-alkyl esters (e.g. the above exemplified compounds in which the tri-substituted silyl groups are substituted with lower alkyl groups (e.g. linear or branched lower alkyl such as methyl, ethyl, n-propyl, isopropyl, n-butyl, tert-butyl); aromatic heterocyclic esters (e.g. pyridyl, pyrimidinyl, pyrazinyl and pyridazinyl esters); lower alkyl esters (e.g. methyl, ethyl, propyl, isopropyl, butyl, isobutyl, tert-butyl, pentyl and hexyl esters), lower alkyl esters which may have at least one suitable substituent, such as lower alkanoyloxy (lower)alkyl esters [e.g. acetoxymethyl, propionyloxymethyl, butyryloxymethyl, valeryloxymethyl, pivaloyloxymethyl, hexanoyloxymethyl, 1-(or 2-)acetoxyethyl, 1-(or 2- or 3-)acetoxypropyl, 1-(or 2-or 3- or 4-)acetoxybutyl, 1-(or 2-)propionyloxyethyl, 1-(or 2- or 3-)propionyloxypropyl, 1-(or 2-)-butyryloxyethyl, 1-(or 2-)isobutyryloxyethyl, 1-(or 2-)-pivaloyloxyethyl, 1-(or 2-)hexanoyloxyethyl, isobutyryloxymethyl, 2-ethylbutyryloxymethyl, 3,3-dimethylbutyryloxymethyl and 1-(or 2-)pentanoyloxyethyl esters], lower alkanesulfonyl (lower)alkyl esters (e.g. 2-mesylethyl ester), mono (or di or tri)halo(lower)alkyl esters (e.g. 2-iodoethyl, 2,2-dichloroethyl and 2,2,2-trichloroethyl esters), lower alkoxycarbonyloxy(lower) alkyl esters [e.g. methoxycarbonyloxymethyl, ethoxycarbonyloxymethyl, propoxycarbonyloxymethyl, tert-butoxycarbonyloxymethyl, 1-(or 2-)-methoxycarbonyloxyethyl, 1- (or 2-) ethoxycarbonyloxyethyl and 1-(or 2 -)isopropoxycarbonyloxyethyl esters], phthalidylidene (lower)alkyl esters, or (5-lower-alkyl-2-oxo-1,3-dioxolane-4-yl) (lower) alkyl esters [e.g. (5-methyl-2-oxo-1,3-dioxolane-4-yl)methyl, (5-ethyl-2-oxo-1,3- dioxolane-4-yl)methyl and (5-propyl-2-oxo-1,3-dioxolane-4-yl) ethyl esters]; lower alkenyl esters (e.g. vinyl and allyl esters); lower alkynyl esters (e.g. ethynyl and propynyl esters); aryl(lower)alkyl esters which may have at least one suitable substituent [e.g. benzyl, 4-methoxybenzyl, 4-nitrobenzyl, phenethyl, trityl, benzhydryl, bis(methoxyphenyl)methyl, 3,4-dimethoxybenzyl and 4-hydroxy-3,5-di--tert-butylbenzyl esters]; aryl esters which may have at least one suitable substituent (e.g. phenyl, 4-chlorophenyl, tolyl, tert-butylphenyl, xylyl, mesityl and cumenyl esters); and phthalidyl esters.

Furthermore, these may be substituted at their individual groups with one or more substituents, being exemplified by halogen atoms such as fluorine, chlorine and bromine atoms; linear or branched lower alkyl groups such as methyl, ethyl, n-propyl, isopropyl, n-butyl, tert-butyl and hexyl groups; monocyclic or polycyclic alkyl groups such as cyclopentyl, cyclohexyl, menthyl, fenchyl and bornyl groups; linear or branched lower alkoxy groups such as methoxy and ethoxy groups; carboxyl group; amino group; nitro group; cyano group; hydroxyl group; aryl groups having 6 to 10 carbon atoms which may be substituted with the above-mentioned halogen atoms and lower alkyl, lower alkoxy, carboxyl, amino, nitro, cyano and hydroxyl groups, such as phenyl, tolyl, xylyl, mesityl and cumenyl; aralkyl groups having 7 to 24 carbon atoms which may be substituted with the above-mentioned halogen atoms and lower alkyl, lower alkoxy, carboxyl, amino, nitro, cyano and hydroxyl groups, such as benzyl, phenethyl, trityl and benzhydryl groups; and the below-described heterocyclic groups, acyl and ester groups.

Preferred examples of the groups represented by X and Y include the groups which can be adopted as synthetic intermediates for carbapenem based antimicrobial compounds, being specifically exemplified by hydrogen atoms and the below-described groups:

Referring to the alkyl, alkenyl, aralkyl, aryl, alkylthio, alkenylthio, aralkylthio, arylthio, alkyloxy, alkenyloxy, aralkyloxy and aryloxy groups, namely, as the alkyl group, there are mentioned linear or branched lower alkyl groups such as methyl, ethyl, n-propyl, isopropyl, n-butyl, tert-butyl and hexyl groups, and monocyclic or polycyclic alkyl groups such as cyclopentyl, cyclohexyl, menthyl, fenchyl and bornyl groups; the alkenyl group includes for example linear or branched lower alkenyl groups such as vinyl, allyl, 1-propenyl, 2-butenyl and 2-methyl-2-propenyl groups; examples of the aralkyl group include aralkyl groups having 7 to 24 carbon atoms such as benzyl, phenethyl, trityl and benzhydryl groups; and as the aryl group, there may be mentioned aryl groups having 6 to 10 carbon atoms such as phenyl, tolyl, xylyl, mesityl and cumenyl groups.

As the silyloxy group, there may be mentioned tri-substituted silyloxy groups, and their specific examples include trialkylsilyloxy, aryl(alkyl)alkoxysilyloxy, alkoxydiarylsilyloxy, triarylsilyloxy, alkyldiarylsilyloxy, aryldialkylsilyloxy and triaralkylsilyloxy groups, being exemplified by trimethylsilyloxy, triethylsilyloxy, triisopropylsilyloxy, dimethylhexylsilyloxy, tert-butyldimethylsilyloxy, methyldiisopropylsilyloxy, isopropyldimethylsilyloxy, tert-butylmethoxyphenylsilyloxy, tert-butoxydiphenyl-silyloxy, triphenylsilyloxy, tert-butyldiphenylsilyloxy, dimethylcumylsilyloxy and tribenzylsilyloxy groups.

These alkyl, alkenyl, aralkyl, aryl, alkylthio, alkenylthio, aralkylthio, arylthio, alkyloxy, alkenyloxy, aralkyloxy, aryloxy and silyloxy groups may be substituted in their individual groups with one or more substituents, for example, halogen atoms such as fluorine, chlorine and bromine atoms; carboxyl group; formyl group; nitro group; cyano group; hydroxyl group; amino group; linear or branched lower alkyl groups such as methyl, ethyl, n-propyl, isopropyl, n-butyl, tert-butyl and hexyl groups; monocyclic and polycyclic alkyl groups such as cyclopentyl, cyclohexyl, menthyl, fenchyl and bornyl groups; linear or branched lower alkenyl groups such as vinyl, allyl, 2-chloroallyl, 1-propenyl, 2-butenyl, and 2-methyl-2-propenyl groups; aryl groups having 6 to 10 carbon atoms such as phenyl, tolyl, xylyl, mesityl and cumenyl groups; aralkyl groups having 7 to 24 carbon atoms such as benzyl, phenethyl, trityl and benzhydroyl groups; alkylthio, alkenylthio, aralkylthio, arylthio, alkyloxy, alkenyloxy, aralkyloxy and aryloxy groups which correspond individually to the above-mentioned alkyl, alkenyl, aralkyl and aryl groups; alkylsulfinyl and alkylsulfonyl groups that correspond individually to the above-mentioned alkyl groups; aralkylsulfinyl and aralkylsulfonyl groups that correspond individually to the above-mentioned aralkyl groups; arylsulfinyl and arylsulfonyl groups that correspond individually to the above-mentioned aryl groups; carbamoyl groups; carbamoyloxy groups; imino-lower-alkyl groups; imino-lower-alkylamino groups; acyloxy groups that correspond individually to the below-described acyl groups; the above-mentioned silyloxy groups; the below-described heterocyclic, heterocyclic-thio, heterocyclic-oxy, acyl, ester, thioester and amide groups.

Furthermore, the above-described substituents individually may be substituted with one or more of substituents such as the above-described substituents: by way of example, the substituents for the alkyl groups (as is the case with the alkylthio and alkyloxy groups) include halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino, imino-lower-alkylamino, acyloxy, silyloxy, heterocyclic, heterocyclic-thio, heterocyclic-oxy, acyl, ester, thioester and amide groups; as the substituents for the alkenyl groups (as is the same with the alkenylthio and alkenyloxy groups), there may be mentioned halogen atoms, and carboxyl, formyl, nitro, cyano, hydroxyl, amino, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; the substituents for the aralkyl groups (as is the same with the aralkylthio and aralkyloxy groups) include for example halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; as the substituents for the aryl groups (as is the same with the arylthio and aryloxy groups), there may be mentioned halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; and the substituents for the amino, carbamoyl, carbamoyloxy, imino-lower-alkyl, imino-lower-alkylamino and amide groups include for example halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups;

The term "heterocyclic group" in the heterocyclic, heterocyclic-thio and heterocyclic-oxy groups is understood to comprehend saturated or unsaturated, monocyclic or polycyclic heterocyclic groups having at least one hetero atom such as oxygen, sulfur and nitrogen atoms, and their preferred examples include 3- to 8-membered, particularly preferably 5- or 6-membered unsaturated monocyclic heterocyclic groups having 1 to 4 nitrogen atoms such as pyrrolyl, pyrrolinyl, imidazolyl, pyrazolyl and pyridyl groups and their N-oxides, pyrimidinyl, pyrazinyl, pyridazinyl, triazolyl (e.g. 4H-1,2,4-triazolyl, 1H-1,2,3-triazolyl and 2H-1,2,3-triazolyl groups), tetrazolyl (e.g. 1H-tetrazolyl and 2H-tetrazolyl groups) and dihydrotriazinyl (e.g. 4,5-dihydro-1,2,4-triazinyl and 2,5-dihydro-1,2,4-triazinyl groups) groups; 3- to 8-membered, particularly preferably 5- or 6-membered saturated monocyclic heterocyclic groups having 1 to 4 nitrogen atoms such as azetidinyl, pyrrolidinyl, imidazolidinyl, piperidyl, pyrazolidinyl and piperazinyl groups; 7- to 12-membered, unsaturated polycyclic heterocyclic groups having 1 to 5 nitrogen atoms such as indolyl, isoindolyl, indolidinyl, benzimidazolyl, quinolyl, isoquinolyl, indazolyl, benzotriazolyl, tetrazopyridyl, tetrazopyridazinyl (e.g. tetrazo[1,5-b]pyridazinyl group) and dihydrotriazolo-pyridazinyl; 3- to 8-membered, particularly preferably 5- or 6-membered unsaturated monocyclic heterocyclic groups having 1 to 2 oxygen atoms and 1 to 3 nitrogen atoms such as oxazolyl, isoxazolyl and oxadiazolyl (e.g. 1,2,4-oxadiazolyl, 1,3,4-oxadiazolyl and 1,2,5-oxadiazolyl groups) groups; 3- to 8-membered, particularly preferably 5- or 6-membered saturated monocyclic heterocyclic groups having 1 to 2 oxygen atoms and 1 to 3 nitrogen atoms such as morpholinyl group; 7- to 12-membered unsaturated polycyclic heterocyclic groups having 1 to 2 oxygen atoms and 1 to 3 nitrogen atoms such as benzoxazolyl and benzoxadiazolyl groups; 3- to 8-membered, particularly preferably 5- or 6-membered unsaturated monocyclic heterocyclic groups having 1 to 2 sulfur atoms and 1 to 3 nitrogen atoms such as 1,3-thiadiazolyl, 1,2-thiazolyl, thiazolinyl and thiadiazolyl (e.g. 1,2,4-thiadiazolyl, 1,3,4-thiadiazolyl, 1,2,5-thiadiazolyl and 1,2,3-thiadiazolyl groups); 3- to 8-membered saturated monocyclic heterocyclic groups having 1 to 2 sulfur atoms and 1 to 3 nitrogen atoms such as thiazolidinyl group; 7-to 12-membered unsaturated polycyclic heterocyclic groups having 1 to 2 sulfur atoms and 1 to 3 nitrogen atoms such as benzothiazolyl and benzothiadiazolyl groups; 3- to 8-membered, particularly preferably 5- or 6-membered unsaturated monocyclic heterocyclic groups having 1 to 2 oxygen atoms such as furanyl and pyranyl groups; 3- to 8-membered, particularly preferably 5- or 6-membered saturated monocyclic heterocyclic groups having 1 to 2 oxygen atoms such as tetrahydrofuranyl and tetrahydropyranyl groups; 3- to 8-membered, particularly preferably 5- or 6-membered unsaturated monocyclic heterocyclic groups having a sulfur atom such as thienyl group and S-oxide; and 3- to 8-membered, particularly preferably 5- or 6-membered saturated monocyclic heterocyclic groups having a sulfur atom such as tetrahydrothienyl group and S-oxide.

These heterocyclic groups may be substituted in their individual groups with one or more substituents, for example, halogen atoms such as fluorine, chlorine and bromine atoms; carboxyl group; formyl group; nitro group; cyano group; hydroxyl group; amino group; linear or branched lower alkyl groups such as methyl, ethyl, n-propyl, isopropyl, n-butyl, tert-butyl and hexyl groups; monocyclic and polycyclic alkyl groups such as cyclopentyl, cyclohexyl, menthyl, fenchyl and bornyl groups; linear or branched lower alkenyl groups such as vinyl, allyl, 2-chloroallyl, 1-propenyl, 2-butenyl, and 2-methyl-2-propenyl groups; aryl groups having 6 to 10 carbon atoms such as phenyl, tolyl, xylyl, mesityl and cumenyl groups; aralkyl groups having 7 to 24 carbon atoms such as benzyl, phenethyl, trityl and benzhydryl groups; alkylthio, alkenylthio, aralkylthio, arylthio, alkyloxy, alkenyloxy, aralkyloxy and aryloxy groups which correspond individually to the above-mentioned alkyl, alkenyl, aralkyl and aryl groups; alkylsulfinyl and alkylsulfonyl groups that correspond individually to the above-mentioned alkyl groups; aralkylsulfinyl and aralkylsulfonyl groups that correspond individually to the above-mentioned aralkyl groups; arylsulfinyl and arylsulfonyl groups that correspond individually to the above-mentioned aryl groups; carbamoyl groups; carbamoyloxy groups; imino-lower-alkyl groups; imino-lower-alkylamino groups; acyloxy groups that correspond individually to the below-described acyl groups; the above-mentioned silyloxy, heterocyclic, heterocyclic-thio, heterocyclic-oxy, acyl, ester, thioester and amide groups.

Furthermore, the above-described substituents individually may be substituted with one or more of substituents such as the above-described substituents: by way of example, the substituents for the alkyl groups (as is the case with the alkylthio and alkyloxy groups) include halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino, imino-lower-alkylamino, acyloxy, silyloxy, heterocyclic, heterocyclic-thio, heterocyclic-oxy, acyl, ester, thioester and amide groups; as the substituents for the alkenyl groups (as is the same with the alkenylthio and alkenyloxy groups), there may be mentioned halogen atoms, and carboxyl, formyl, nitro, cyano, hydroxyl, amino, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; the substituents for the aralkyl groups (as is the same with the aralkylthio and aralkyloxy groups) include for example halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; as the substituents for the aryl groups (as is the same with the arylthio and arylbxy groups), there may be mentioned halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; and the substituents for the amino, carbamoyl, carbamoyloxy, imino-lower-alkyl, imino-lower-alkylamino and amide groups include, for example, halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups.

More specific examples include:

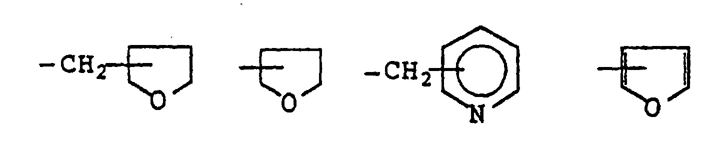

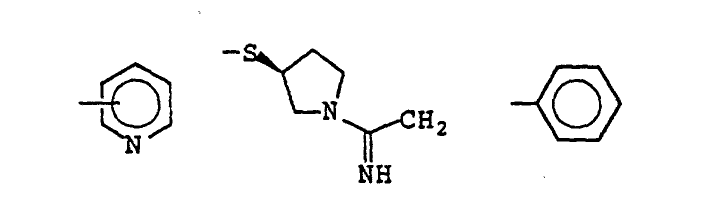

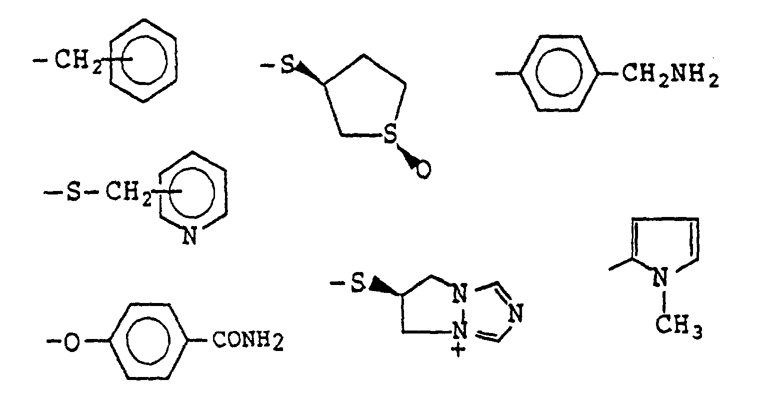

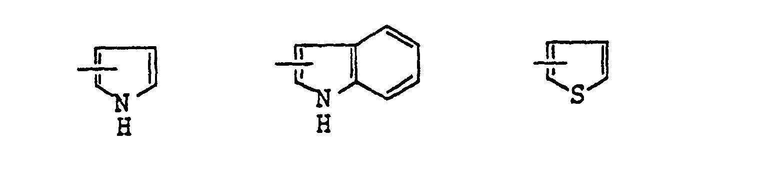

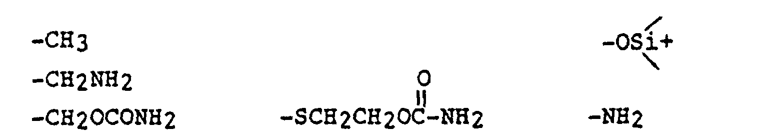

- SCH₂CH₃

-SCH₂CH₂NHCH=NH

- SCH₂CH₂NH₂

-CONH₂

and acyl groups containing them (which have the carbonyl group attached to the linkages).

As the acyl group, there may be mentioned alkylcarbonyl, alkenylcarbonyl, aralkylcarbonyl, arylcarbonyl, alkylthiocarbonyl, alkenylthiocarbonyl, aralkylthiocarbonyl, arylthiocarbonyl, alkyloxycarbonyl, alkenyloxycarbonyl, aralkyloxycarbonyl, aryloxycarbonyl, silyloxycarbonyl, heterocyclic carbonyl, heterocyclic thiocarbonyl, heterocyclic oxycarbonyl, estercarbonyl, thioestercarbonyl and amidocarbonyl groups which correspond individually to the above-mentioned alkyl, alkenyl, aralkyl, aryl, alkylthio, alkenylthio, aralkylthio, arylthio, alkyloxy, alkenyloxy, aralkyloxy, aryloxy, silyloxy, heterocyclic, heterocyclic-thio and heterocyclic-oxy groups, as well as the below-described ester, thioester and amide groups.

As the ester and thioester groups, furthermore, there may be mentioned carboxyl and thiocarboxyl groups which are esterified with the above-mentioned alkyl, alkenyl, aralkyl, aryl, alkylthio, alkenylthio, aralkylthio, arylthio, alkyloxy, alkenyloxy, aralkyloxy, aryloxy, silyl, heterocyclic, heterocyclic-thio and heterocyclic-oxy or acyl groups, as well as the below-described amide and amino groups, while examples of the amide and amino groups include amide and amino groups which may be substituted individually, for example with one or more of the above-mentioned substituents for the amide and amino groups. As the halogen atom, there may be mentioned fluorine, chlorine and bromine atoms.

Referring to the cycloalkane-2-on-1-yl groups, which X and Y together represent, examples of such cycloalkanes include monocyclic ones such as cyclopentane and cyclohexane, which may be substituted individually, for example with one or more of the above-mentioned substituents for the alkyl groups.

As the groups represented by X', there may be mentioned the groups represented by X as well as mercapto, hydroxyl, formyl, carboxyl and thiocarboxyl groups.

The groups represented by R₄ are not particularly limited, only if they can be adopted as a synthetic intermediate for the carbapenem based antimicrobial compounds, and include for example hydrogen atom or the protective or substituent groups for the amino group to be described in the following:

Namely, preferred examples of the protective and substituent groups for the amino group include alkyl, alkenyl, aralkyl, aryl, acyl, amide and silyl groups and halogen atoms, wherein as the alkyl groups, there may be mentioned linear or branched lower alkyl groups such as methyl, ethyl, n-propyl, isopropyl, n-butyl, tert-butyl and hexyl groups and monocyclic or polycyclic alkyl groups such as cyclopentyl, cyclohexyl, menthyl, fenchyl and bornyl groups; examples of the alkenyl groups include linear or branched lower alkenyl groups such as vinyl, allyl, 1-propenyl, 2-butenyl and 2-methyl-2-propenyl groups; as the aralkyl groups, there may be mentioned, for example, aralkyl groups having 7 to 24 carbon atoms such as benzyl, phenethyl, trityl and benzhydryl groups; and examples of the aryl groups include aryl groups having 6 to 10 carbon atoms such as phenyl, tolyl, xylyl, mesityl and cumenyl groups.

As the silyl groups, there may be mentioned tri-substituted silyl groups, which specifically include trialkylsilyl, aryl (alkyl)alkoxysilyl, alkoxydiarylsilyl, triarylsilyl, alkyldiarylsilyl, aryldialkylsilyl and triaralkylsilyl groups, such as trimethylsilyl, triethylsilyl, triisopropylsilyl, dimethylhexylsilyl, tert-butyldimethylsilyl, methyldiisopropylsilyl, isopropyldimethylsilyl, tert-butylmethoxyphenylsilyl, tert-butoxydiphenylsilyl, triphenylsilyl, tert-butyldiphenylsilyl, dimethylcumylsilyl and tribenzylsilyl groups.

These alkyl, alkenyl, aralkyl, aryl and silyl groups may be substituted in their individual groups with one or more substituents, for example, halogen atoms such as fluorine, chlorine and bromine atoms; carboxyl groups; formyl groups; nitro group; cyano group; hydroxyl group; amino groups; linear or branched lower alkyl groups such as methyl, ethyl, n-propyl, isopropyl, n-butyl, tert-butyl and hexyl groups; monocyclic or polycyclic alkyl groups such as cyclopentyl, cyclohexyl, menthyl, fenchyl and bornyl groups; linear or branched lower alkenyl groups such as vinyl, allyl, 2-chloroallyl, 1-propenyl, 2-butenyl and 2-methyl-2-propenyl groups; aryl groups having 6 to 10 carbon atoms such as phenyl, tolyl, xylyl, mesityl and cumenyl groups; aralkyl groups having 7 to 24 carbon atoms such as benzyl, phenethyl, trityl and benzhydryl groups; alkylthio, alkenylthio, aralkylthio, arylthio, alkyloxy, alkenyloxy, aralkyloxy and aryloxy groups which correspond individually to the above-mentioned alkyl, alkenyl; aralkyl and aryl groups; alkylsulfinyl and alkylsulfonyl groups that correspond individually to the above-mentioned alkyl groups; aralkylsulfinyl and aralkylsulfonyl groups that correspond individually to the above-mentioned aralkyl groups; arylsulfinyl and arylsulfonyl groups that correspond individually to the above-mentioned aryl groups; carbamoyl groups; carbamoyloxy groups; imino-lower-alkyl groups; imino-lower-alkylamino groups; acyloxy groups that correspond individually to the below-described acyl groups; the above-mentioned silyloxy groups; the above-described heterocyclic, heterocyclic-thio, heterocyclic-oxy, acyl, ester, thioester and amide groups.

Furthermore, the above-described substituents individually may be substituted with one or more substituents such as the above-described substituents: by way of example, the substituents for the alkyl groups (as is the same with the alkylthio and alkyloxy groups) include halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino, imino-lower-alkylamino, acyloxy, silyloxy, heterocyclic, heterocyclic-thio, heterocyclic-oxy, acyl, ester, thioester and amide groups; as the substituents for the alkenyl groups (as is the same with the alkenylthio and alkenyloxy groups), there may be mentioned halogen atoms, and carboxyl, formyl, nitro, cyano, hydroxyl, amino, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio/ heterocyclic-oxy, acyl, ester, thioester and amide groups; the substituents for the aralkyl groups (as is the same with the aralkylthio and aralkyloxy groups) include for example halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; as the substituents for the aryl groups (as is the same with the arylthio and aryloxy groups), there may be mentioned halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups; and the substituents for the amino, carbamoyl, carbamoyloxy, imino-lower-alkyl, imino-lower-alkylamino and amide groups include for example halogen atoms, and carboxyl, formyl, nitro, cyano, hydroxyl, amino, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl, carbamoyloxy, imino-lower-alkyl, imino-lower-alkylamino, acyloxy, silyloxy, heterocyclic, heterocyclic-thio, heterocyclic-oxy, acyl, ester, thioester and amide groups.

As the acyl group, there may be mentioned alkylcarbonyl, alkenylcarbonyl, aralkylcarbonyl, arylcarbonyl, alkylthiocarbonyl, alkenylthiocarbonyl, aralkylthiocarbonyl, arylthiocarbonyl, alkyloxycarbonyl, alkenyloxycarbonyl, aralkyloxycarbonyl, aryloxycarbonyl, silyloxycarbonyl, heterocyclic carbonyl, heterocyclic thiocarbonyl, heterocyclic oxycarbonyl, estercarbonyl, thioestercarbonyl and amidocarbonyl groups which correspond individually to the above-mentioned alkyl, alkenyl, aralkyl, aryl, alkylthio, alkenylthio, aralkylthio, arylthio, alkyloxy, alkenyloxy, aralkyloxy, aryloxy, silyloxy, heterocyclic, heterocyclic-thio and heterocyclic-oxy, ester and thioester groups as well as the below-described amide groups.

As the amide groups, furthermore, there may be mentioned amide groups which individually may be substituted with one or more substituents such as the above-mentioned substituents for the amide groups, and examples of the halogen atoms include fluorine, chlorine and bromine atoms.

The protective groups for the amino group are not particularly limited, and there may be suitably selected and used conventionally employed protective groups, preferred examples of which are exemplified by the above-mentioned silyl groups; lower-alkoxy lower alkyl groups which may have at least one suitable substituent, such as methoxymethyl and methoxyethoxymethyl groups; aralkyloxycarbonyl-lower-alkyl groups which may have at least one suitable substituent, such as benzyloxycarbonylmethyl, p-nitrobenzyloxycarbonylmethyl, p-methoxybenzyloxycarbonylmethyl, phenethyloxycarbonylmethyl, trityloxycarbonylmethyl, benzhydryloxycarbonylmethyl, bis(methoxyphenyl)methyloxycarbonylmethyl, 3,4-dimethoxybenzyloxycarbonylmethyl and 4-hydroxy-3,5-di-tert-butylbenzyloxycarbonylmethyl groups; aralkyl groups which may have at least one suitable substituent, such as benzyl, p-methoxybenzyl, p-nitrobenzyl, p-tert-butylbenzyl, 3,4-dimethylbenzyl, phenethyl, benzhydryl, trityl, bis(methoxyphenyl)-methyl, 2,4-dimethoxybenzyl, 3,4-dimethoxybenzyl, 4-hydroxy-3,5-di-tert-butylbenzyl and 2-naphthylmethyl groups; aralkylcarbonyl groups which may have at least one suitable substituent, such as benzylcarbonyl, p-methoxybenzylcarbonyl, p-nitrobenzylcarbonyl, p-tert-butylbenzylcarbonyl, 3,4-dimethylbenzylcarbonyl, phenethylcarbonyl, benzhydrylcarbonyl, tritylcarbonyl, bis (methoxyphenyl)-methylcarbonyl, 2,4-dimethoxybenzylcarbonyl, 3,4-dimethoxybenzylcarbonyl, 4-hydroxy-3,5-di-tert-butylbenzylcarbonyl and 2-naphthylmethylcarbonyl groups; heterocyclic groups which may have at least one suitable substituent, such as tetrahydropyranyl group; arylcarbonyl groups which may have at last one suitable substituent, such as benzoyl, chlorobenzoyl, p-methoxybenzoyl, p-nitrobenzoyl, p-tert-butylbenzoyl, toluoyl and naphthoyl groups; arylcarbonyl-lower-alkyl groups which may have at least one suitable substituent, such as benzoylmethyl, chlorobenzoylmethyl, p-methoxybenzoylmethyl, p-nitrobenzoylmethyl, p-tert-butylbenzoylmethyl, toluoylmethyl and naphthoylmethyl groups; aryloxy-lower-alkylcarbonyl groups which may have at least one suitable substituent, such as phenoxyacetyl, 4-chlorophenoxyacetyl, tolyloxyacetyl, tert-butylphenoxyacetyl, xylyloxyacetyl, mesityloxyacetyl and cumenyloxyacetyl groups; arylsulfonyl groups which may have at least one suitable substituent, such as benzenesulfonyl, p-tert-butylbenzenesulfonyl and toluenesulfonyl groups; alkylsulfonyl groups which may have at least one suitable substituent, such as mesyl group; formyl group; aliphatic carboxylic acid acyl groups which may have at least one suitable substituent, such as acetyl, chloroacetyl, bromoacetyl, dichloroacetyl, trichloroacetyl, methoxyacetyl, propionyl, butyryl, isobutyryl, valeryl, pivaloyl, hexanoyl, 2-ethylbutyryl, 3,3-dimethylbutyryl, pentanoyl, caprylyl, decanoyl and acryloyl groups; lower-alkoxycarbonyl groups which may have at least one suitable substituent, such as methoxycarbonyl, ethoxycarbonyl, propoxycarbonyl, isopropoxycarbonyl, tert-butoxycarbonyl, 2-iodoethoxycarbonyl, 2,2-dichloroethoxycarbonyl and 2,2,2-trichloroethoxycarbonyl groups; lower-alkenyloxycarbonyl groups which may have at least one suitable substituent, such as vinyloxycarbonyl, allyloxycarbonyl and 2-chloroallyloxycarbonyl groups; aralkyloxycarbonyl groups which may have at least one suitable substituent, such as benzyloxycarbonyl, p-nitrobenzyloxycarbonyl, benzhydryloxycarbonyl and trityloxycarbonyl groups; aryloxycarbonyl groups which may have at least one suitable substituent, such as phenoxycarbonyl, 4-chlorophenoxycarbonyl and tert-butylphenoxycarbonyl groups; and carbamoyl groups which may have at least one suitable substituent and their corresponding thiocarbamoyl groups, such as methylcarbamoyl, phenylcarbamoyl and naphthylcarbamoyl groups.

Below described are Examples to illustrate the present invention in more detail, but the invention is not limited to these examples.

### Example 1

### Synthesis of (3S,4S)-3-[(R )-1-(tert-butyldimethylsilyloxy)-ethyl]-4-di(methoxycarbonyl)methyl-azetidinone

A solution of dimethyl malonate (291 mg, 2.2 mmole) in tetrahydrofuran (5 ml) was added to a suspension of sodium hydride (90 mg, 2.2 mmole) in tetrahydrofuran (10 ml) in a nitrogen stream, while stirring under ice cooling, and the solution mixture was stirred for 10 min. and then admixed with cuprous bromide dimethylsulfide complex (452 mg, 2.2 mmole), followed by stirring for 15 min, addition of a solution of (3S,4R) -3-[(R ) -1-(tert-butyldimethylsilyloxy)-ethyl]-4-phenylthio-2-azetidinone (338 mg, 1 mmole) in tetrahydrofuran (10 ml) and stirring for 15 min. The reaction solution was admixed with saturated ammonium chloride solution, and after the insoluble matter was removed by filtration, the filtrate was extracted with ethyl acetate. The ethyl acetate layer was washed with aqueous saturated sodium chloride solution three times, dried over magnesium sulfate and concentrated under reduced pressure, and the residue was purified by silica gel column chromatography (the developing solvent was a 7:1 mixture of n-hexane and ethyl acetate) to give 267 mg (74.4 %) of the subject compound in the form of a colorless solid.

IR (KBr) cm⁻¹: 3158, 3103, 2958, 1770, 1774, 1440, 1202, 836. NMR (CDCl₃): δ 0.07(6H,s, -Si(CH₃)₂), 0.88(9H,s, -SiC(CH₃)₃), 1.11(3H,d,J=6.6Hz, CH₃CH-OSi), 3.04(1H,m, C3-H), 3.55(1H,d,J=9.2Hz, CH(CO₂CH₃)₂), 3.77 and 3.78 (each 3H,s, CO₂CH₃), 4.18-4.28(1H,m,CH₃CH-OSi) , 6.06(1H,brs,-NH).

### Example 2

### Synthesis of (3S,4S)-3-[(R)-1-(tert-butyldimethylsilyloxy)-ethyl)-4-[1,1-di(ethoxycarbonyl)ethyl-2-azetidinone

A solution of diethyl 2-methylmalonate (192 mg, 1.1 mmole) in tetrahydrofuran (2.5 ml) was added to a suspension of sodium hydride (44 mg, 1.1 mmole) in tetrahydrofuran (5 ml) in a nitrogen stream, while stirring under ice cooling, and the solution mixture was stirred for 10 min. and then admixed with cuprous bromide dimethylsulfide complex (227 mg, 1.1 mmole), followed by stirring for 15 min, addition of a solution of (3S,4R)-3-[(R)-1-(tert-butyldimethylsilyloxy)ethyl]-4-phenylthio-2-azetidinone (169 mg, 0.5 mmole) in tetrahydrofuran (5 ml) and stirring for 15 min. The reaction solution was admixed with saturated ammonium chloride solution, and after the insoluble matter was removed by filtration, the filtrate was extracted with ethyl acetate. The ethyl acetate layer was washed with aqueous saturated sodium chloride solution three times, dried over magnesium sulfate and concentrated under reduced pressure, and the residue was purified by silica gel column chromatography (the developing solvent was a 7:1 mixture of n-hexane and ethyl acetate) to give 106 mg (52.8 %) of the subject compound in the form of a colorless solid.

IR (KBr) cm⁻¹: 3178, 2856, 1769, 1756, 1736, 1256, 1114, 837. NMR (CDCl₃): δ 0.07(6H,s,-Si(CH₃)₂), 0.88(9H, s,-SiC(CH₃)₃), 1.14(3H,d,J=5.9Hz, CH₃CH-OSi), 1.23-1.31 (6H,m, (CO₂CH₂CH₃)₂), 1.46(3H,s,CCH₃(CO₂CH₂CH₃)₂), 3.01 (1H,d,J=1.92Hz,C4-H), 4.12-4.28 (6H,m,CH₃CH-OSi, -OSi, C3-H and (CO₂CH₂CH₃)₂), 5,97 (1H,brs,-NH).

### Example 3

### Synthesis of (3S, 4S)-3-[(R)-1-(tert-butyldimethylsilyloxy)ethyl]-4-di(ethoxycarbonyl)fluoromethyl-2-azetidinone

A solution of diethyl 2-fluoromalonate (196 mg, 1.1 mmole) in tetrahydrofuran (2.5 ml) was added to a suspension of sodium hydride (44 mg, 1.1 mmole) in tetrahydrofuran (5 ml) in a nitrogen stream, while stirring under ice cooling, and the solution mixture was stirred for 10 min. and then admixed with cuprous bromide dimethylsulfide complex (227 mg, 1.1 mmole), followed by stirring for 15 min, addition of a solution of (3S,4R)-3-[(R)-1-(tert-butyldimethylsilyloxy)ethyl]-4-phenylthio-2-azetidinone (169 mg, 0.5 mmole) in tetrahydrofuran (5 ml) and stirring for 15 min. The reaction solution was admixed with saturated ammonium chloride solution, and after the insoluble matter was removed by filtration, the filtrate was extracted with ethyl acetate. The ethyl acetate layer was washed with aqueous saturated sodium chloride solution three times, dried over magnesium sulfate and concentrated under reduced pressure, and the residue was purified by silica gel column chromatography (the developing solvent was a 7:1 mixture of n-hexane and ethyl acetate) to give 166 mg (82.%) of the subject compound in the form of a colorless solid.

IR (KBr) cm⁻¹: 3169, 3104, 2854, 1744, 1473, 1289, 1251, 836. NMR (CDCl₃) : δ 0.07(6H,s,-Si(CH₃)₂), 0.88(9H,s, -SiC(CH₃)₃), 1.08(3H,d,J=5.9Hz, CH₃CH-OSi), 1.30-1.36 (6H,m,(CO₂CH₂CH₃)₂), 3.30(1H,s,C4-H), 4.24-4.45(6H,m, CH₃CH-OSi, C3-H and (CO₂CH₂CH₃)₂), 5.94(1H,brs,-NH).

### Example 4

### Synthesis of (3S,4S)-3-[(R)-1-(tert-butyldimethylsilyloxy)ethyl]-4-(hydroxycarbonyl)fluoromethyl-2-azetidinone

(3S,4S)-3-[(R)-1- (tert-butyldimethylsilyloxy) ethyl]--4-di(ethoxycarbonyl)-fluoromethyl-2-azetidinone (405 mg, 1.0 mmole) was dissolved in 1 ml of pyridine, and 0.5 ml of 4N sodium hydroxide solution was added to the solution, followed by stirring at room temperature for 1.5 hours. The reaction solution was admixed with 0.4 ml of 5N hydrochloric acid and 2.0 ml of 2,4,6-collidine, followed by heating at 150°C and stirring for 1 hour. After the reaction solution was allowed to cool, the solvent was distilled off, and the residue was dissolved in chloroform. The solution was washed with aqueous saturated potassium hydrogen sulfate solution twice, admixed with aqueous saturated sodium hydrogen carbonate solution and separated into two layers and the aqueous layer was washed with chloroform once, admixed with potassium hydrogen sulfate and sodium chloride, and extracted with ethyl acetate. The ethyl acetate layer was washed with aqueous saturated sodium chloride solution twice, dried over magnesium sulfate and concentrated under reduced pressure, and the residue was recrystallized from ethyl acetate/hexane to give 254 mg (83.%) of the subject compound consisting of a diastereomer mixture at a ratio of about 2:1 in the form of a colorless solid.

IR (KBr) cm⁻¹: 3406, 2929, 1748, 1473, 1376, 1254, 1168, 1143, 1112, 1072, 1041, 962, 839, 780. NMR (DMSO-d₆): Major product: δ 0.02 and 0.05(each 3H,s, -Si(CH₃)₂), 0.84(9H,s,-SiC(CH₃)₃), 1.05(3H,d,J=5.9Hz, CH₃CH-OSi), 3.08(1H,m,C3-H), 3.88(1H,m,J=21Hz,C4-H), 4.13(1H,m,CH₃CH-OSi), 5.20(1H,dd,J=49Hz,2.6Hz,F-CH-COOH), 8.11(1H,brs,-NH), 13.62(1H,brs,-COOH). By-product: δ 0.03 and 0.05(each 3H,s,-Si(CH₃)₂), 0.84(9H,s,-SiC(CH₃)₃) , 1.11 (3H,d,J=6.6Hz, CH₃CH-OSi), 3.08(1H,m,C3-H), 3.88(1H,m, J=21Hz,C4-H), 4.13(1H,m,CH₃CH-OSi), 5.08(1H,dd,J=48Hz, 4.6Hz,F-CH-COOH), 8.29(1H,brs,-NH), 13.62(1H,brs,-COOH). MS(FAB) m/z = 306(C₁₃H₂₄NO₄FSi+H)⁺.

### Example 5

### Synthesis of (3S,4S)-3-[(R)-1-[(tert-butyldimethylsilyloxy)-ethyl]-4-(1-acetyl-1-ethoxycarbonylethyl)-2-azetidinone

Ethyl 2-methylacetoacetate (0.87 ml, 6.03 mmole) was added to a suspension of sodium hydride (240 mg, 6.0 mmole) in tetrahydrofuran (5 ml) in a nitrogen stream, and the solution mixture was stirred for 10 min at room temperature and admixed with cuprous bromide dimethylsulfide complex (1.23 g, 5.98 mmole), followed by stirring for 1 hour, addition of (3S,4R)-3-[(R)-1-(tert-butyldimethylsilyloxy) ethyl]-4-phenylthio-2-azetidinone (1.01 g, 2.99 mmole) and stirring for 3 hours. The reaction solution was admixed with saturated ammonium chloride solution, and after the insoluble matter was removed by filtration, the filtrate was extracted with ethyl acetate. The ethyl acetate layer was washed with aqueous saturated sodium chloride solution three times, dried over magnesium sulfate and concentrated under reduced pressure, and the residue was purified by silica gel column chromatography (the developing solvent was a 4:1 mixture of n-hexane and ethyl acetate) to give 876 mg (78.9 %) of the subject compound consisting of a diastereomer mixture at a ratio of about 5:4 in the form of a colorless solid.

IR (KBr) cm⁻¹: 3178, 2957, 2929, 2856, 1767, 1744, 1716, 1646, 1472, 1377, 1362, 1340, 1250, 1159, 1105, 1063, 967, 837, 777. NMR (CDCl₃): Major product: δ 0.06(6H,s,-Si(CH₃)₂), 0.88(9H,s,-SiC(CH₃)₃), 1.19 (3H,d,J=5.9Hz, CH₃CH-OSi), 1.27(3H,t,J=7.2Hz,CO₂CH₂CH₃) , 1.42(3H,s, CCH₃(CO₂CH₂CH₃)(COCH₃)), 2.21(3H,s,CCH₃(CO₂CH₂CH₃) (COCH₃)), 2.91 (1H,m,C3-H), 4.03(1H,d,J=2.0Hz,C4-H), 4.16-4.26(3H,m, CH₃CH-OSi and (CO₂CH₂CH₃), 5.89(1H,brs,-NH). By-product: δ 0.07 (6H,s,-Si(CH₃)₂), 0.88(9H,s,-SiC(CH₃)₃), 1.06(3H,d, J=6.6Hz, CH₃CH-OSi), 1.31(3H,t,J=7.2Hz, CO₂CH₂CH₃), 1.39(3H,s,CCH₃(CO₂CH₂CH₃)COCH₃)), 2.18(3H,s, CCH₃(CO₂CH₂CH₃)(COCH₃) ) , 2.93 (1H,m,C3-H), 4.16-4.26(4H,m, CH₃CH-OSi,C4-H and CO₂CH₂CH₃), 5.89(1H,brs,-NH).

### Example 6

### Synthesis of (3S,4S) -3- [(R)-1-(tert-butyldimethylsilyloxy)- ethyl]-4- (1-acetyl-1-allyloxycarbonylethyl -2-azetidinone

Allyl 2-methylacetoacetate (624 mg, 4.00 mmole) was added to a suspension of sodium hydride (160 mg, 4.0 mmole) in tetrahydrofuran (10 ml) in a nitrogen stream, and the solution mixture was stirred for 10 min at room temperature and then admixed with cuprous bromide dimethylsulfide complex (822 mg, 5.98 mmole), followed by stirring for 1 hour, addition of (3S,4R)-3-[(R)-1-(tert-butyldimethylsilyloxy)ethyl]-4-phenylthio-2-azetidinone (677 mg, 2.00 mmole) and stirring for 3 hours. The reaction solution was admixed with saturated ammonium chloride solution, and after the insoluble matter was removed by filtration, the filtrate was extracted with ethyl acetate. The ethyl acetate layer was washed with aqueous saturated sodium chloride solution three times, dried over magnesium sulfate and concentrated under reduced pressure, and the residue was purified by silica gel column chromatography (the developing solvent was a 3:1 mixture of n-hexane and ethyl acetate) to give 495 mg (64.5%) of the subject compound consisting of a diastereomer mixture (Products A and B) at a ratio of about 1:1 in the form of a colorless solid.

IR (KBr) cm-1: 3178, 2956, 2929, 2857, 1769, 1715, 1250, 1158, 1104, 1063, 966, 838, 778. NMR (CDCl₃): Product A: δ 0.06(6H,s,-Si(CH₃)2), 0.88(9H,s,-SiC(CH₃)₃), 1.19 (3H,d,J=6.6Hz, CH₃CH-OSi), 1.44(3H,s, CCH₃(CO₂)(COCH₃)), 2.21(3H,s,CCH₃(CO₂)(COCH₃)), 2.92(1H,m, C3-H), 4.15-4.24(1H,m,CH₃CH-OSi), 4.25(1H,d,J=2.OHZ,C4-H), 4.62-4.69 (2H,m,OCH₂CHCH₂) , 5.28-5.40 (2H,m,OCH₂CHCH₂), 5.83-5.93(2H,m,-NH and OCH₂CHCH₂).
Product B: δ 0.06 (6H,s,-Si(CH₃)2), 0.88(9H,s,-Si(CH₃)₃), 1.06(3H,d,J=5.9Hz, CH₃CH-OSi), 1.40(3H,s,CCH₃(CO₂)(COCH₃)), 2.18 (3H,s, CCH₃(CO₂)(COCH₃)), 2.94 (1H,m,C3-H), 4.05(1H,d, J=2.6Hz, C4-H), 4.15-4.24(1H,m,CH₃CH-OSi) 4.62-4.69(2H,m, OCH₂CHCH₂), 5.28-5.40(2H,m,OCH₂CHCH₂), 5.83-5.93(2H,m,-NH and OCH₂CHCH₂)

### Example 7

### Synthesis of (3S,4S)-3-[(R)-1-(tert-butyldimethylsilyloxy)-ethyl]-4-(1-acetyl-1-benzyloxycarbonylethyl)-2-azetidinone

Benzyl 2-methylacetoacetate (825 mg, 4.00 mmole) was added to a suspension of sodium hydride (160 mg, 4.0 mmole) in tetrahydrofuran (10 ml) in a nitrogen stream, and the solution mixture was stirred for 10 min at room temperature and then admixed with cuprous bromide dimethylsulfide complex (822 mg, 5.98 mmole), followed by stirring for 1 hour, addition of (3S, 4R)-3-[(R )-1-(tert-butyldimethylsilyloxy) ethyl]-4-phenylthio-2-azetidinone (677 mg, 2.00 mmole) and stirring for 3 hours. The reaction solution was admixed with saturated ammonium chloride solution, and after the insoluble matter was removed by filtration, the filtrate was extracted with ethyl acetate. The ethyl acetate layer was washed with aqueous saturated sodium chloride solution three times, dried over magnesium sulfate and concentrated under reduced pressure, and the residue was purified by silica gel column chromatography (the developing solvent was a 3:1 mixture of n-hexane and ethyl acetate) to give 673 mg (77.6 %) of the subject compound consisting of a diastereomer mixture (Products A and B) at a ratio of about 1:1 in the form of a colorless oily matter.

IR (neat) cm⁻¹: 3259, 2955, 2930, 2856, 1770, 1713, 1498, 1462, 1455, 1373, 1360, 1253, 1142, 1104, 1067, 960, 837, 778, 695. NMR (CDCl₃):
Product A: δ 0.05(6H,s,-Si(CH₃)₂), 0.87(9H,s,-SiC(CH₃)₃), 1.03 (3H,d,J=6.6Hz, CH₃CH-OSi), 1.39(3H,s, CCH₃(CO₂) (COCH₃)), 2. 04(3H, s, CCH₃(CO₂)-(COCH₃)), 2.92(1H,m,C3-H), 4.11-4.22(1H,m,CH₃CH-OSi), 4.27(1H,d, J=2.0Hz,C4-H), 5.10-5.28( 2H,m,OCH₂C₆H₅) , 5.83(1H,brs,-NH), 7.30-7.38(5H,m,C₆H₅). Product B: δ 0.05 and 0.07(each 3H, s,-Si(CH₃)₂), 0.88(9H,s,-SiC(CH₃)3), 1.17(3H,d,J=6.6Hz, CH₃CH-OSi), 1.43(3H,s,CCH₃(CO₂), (COCH₃) ) , 2.12(3H,s, CCH₃(CO₂)(COCH₃)), 2.93(1H,m,C3-H), 4.06(1H,d, J=2.0Hz, C4-H), 4.11-4.22(1H,m,CH₃CH-OSi), 5.10-5.28 (2H,m, OCH₂C₆H₅), 5.83(1H,brs,-NH), 7.30-7.38(5H,m,C₆H₅).

### Example 8

### Synthesis of (3S,4S)-3-[(R)-1-(tert-butyldimethylsilyloxy)ethyl]-4-(1-acetylethyl)-2-azetidinone

(3S,4S)-3-[(R)-1-(tert-butyldimethylsilyloxy)ethyl]-4-(1-acetyl-1-allyloxycarbonylethyl)-2-azetidinone (192 mg, 0.50 mmole) was dissolved in tetrahydrofuran (10 ml) under a nitrogen stream, and dimedone (70 mg, 0.50 mmole) and tetrakis(triphenylphosphine)palladium (58 mg, 0.05 mmole) were added to the resultant solution, followed by stirring under reflux heating for 3 hours. The reaction solution was concentrated under reduced pressure, and the residue was purified by silica gel column chromatography (the developing solvent was a 1:1 mixture of n-hexane and ethyl acetate) to give 108 mg (72.0 %) of the subject compound consisting of a diastereomer at a ratio of about 5:1 in the form of a colorless solid.

IR (KBr) cm⁻¹: 3314, 2954, 1717, 1713, 1654, 1617, 1437, 1308, 1276, 1170, 840. NMR (CDCl₃) : Major product: δ 0.07 and 0.08(each 3H,s,-Si(CH₃)₂), 0.88(9H,s, -SiC(CH₃)₃), 1.23 (3H,d,J=7.3Hz, CHCH₃(COCH₃)), 1.25(3H,d, J=6.0Hz, CH₃CH-OSi), 2.20(3H,s,CHCH₃(COCH₃)), 2.58(1H,dq, J=9.9Hz and 7.6Hz,CHCH₃(COCH₃)), 2.72(1H,dd,J=1.3Hz and 5.9Hz,C3-H) , 3.67(1H,dd,J=2.OHz and 9.9Hz,C4-H), 4.16(1H,m, CH₃CH-OSi), 5.98(1H,brs,-NH).
By-product B: δ 0.07(6H,s,-Si(CH₃)₂), 0.88(9H,s, -SiC(CH₃)₃), 1.18(3H,d,J=7,2Hz,CHCH₃(COCH₃)), 1.20(3H,d, J=6.6Hz, CH₃CH-OSi), 2.22(3H,s,CHCH₃(COCH₃)), 2.81(1H,m,
CHCH₃(COCH₃)), 2.87(1H,dd,J=2.0Hz and 5.3Hz,C3-H), 3.89(1H, dd,J=2.0Hz and 4.6Hz,C4-H), 4.16 (1H, m, CH₃CH-OSi) , 5.92(1H, brs,-NH), MS(FAB) m/z = 300(C₁₅H₂₉NO₃Si+H)⁺.

### Example 9

### Synthesis of (3S,4S)-3-[(R)-1-(tert-butyldimethylsilyloxy)-ethyl]-4-ethoxycarbonylmethyl-2-azetidinone

A mixture of (3S,4R)-3-[(R )-1-(tert-butyldimethylsilyloxy)ethyl]-4-phenylthio-2-azetidinone (338 mg, 1 mmole), zinc (260 mg, 4mg atom), cuprous bromide dimethylsulfide complex (411 mg, 2 mmole), ethyl bromoacetate (0.23 ml, 2 mmole) and tetrahydrofuran (4.5 ml) was stirred at room temperature for 31 hours. The reaction mixture was admixed with saturated ammonium chloride solution and extracted with ethyl acetate, and the ethyl acetate layer was washed with aqueous saturated sodium chloride solution three times, dried over magnesium sulfate and concentrated under reduced pressure. The residue was purified by silica gel column chromatography (the developing solvent was a 7:1 mixture of n-hexane and ethyl acetate) to give 90 mg (29 %) of the subject compound in the form of a colorless solid.

IR (KBr) cm⁻¹: 3186, 2954, 1761, 1729, 1472, 1248, 1140. NMR (CDCl₃): δ 0. 08(6H,s,-Si(CH₃)₂), 0.88(9H, s,-SiC(CH₃)₃), 1.21(3H,d,J=6.6Hz, CH₃CH-OSi), 2.56(1H,dd, J=9,9Hz and 16.5Hz,-CH₂CO₂-), 2.73(1H,dd,J=4.0Hz and 16.5Hz,-CH₂CO₂-), 2.82(1H,dd,J=2.0Hz and 4. 6Hz, C3-H), 3.97(1H,m,C4-H), 4.13-4.24(3H,m,CH₃CH-OSi), 6.15(1H,brs,-NH).

### Example 10

### Synthesis of (3S,4S)-3-[(R)-1-(tert-butyldimethylsilyloxy)-ethyl]-4-[1,1-di(allyloxycarbonyl)ethyl]-2-azetidinone

A solution of diallyl 2-methylmalonate (396 mg, 2 mmole) in tetrahydrofuran (2 ml) was added dropwise to a suspension of sodium hydride (80 mg, 2 mmole) in tetrahydrofuran (5 ml) under an argon stream at 0°C, and the solution mixture was stirred for 5 min and admixed with cuprous bromide dimethylsulfide complex (411 mg, 2 mmole), followed by stirring for 30 min at the same temperature, addition of a solution of (3R,4S)-3-[(R)-1-(tert-butyldimethylsilyloxy) ethyl]-4-phenylthio-2-azetidinone (338 mg, 1 mmole) in tetrahydrofuran (3 ml) and stirring for 1 hour. The reaction solution was admixed with aqueous saturated ammonium chloride solution and extracted with ethyl acetate. The ethyl acetate layer was dried over magnesium sulfate and concentrated under reduced pressure, and the residue was purified by column chromatography (10 g of silica gel; a 8/1 mixture of n-hexane/ethyl acetate) to give 392 mg (92 %) of the subject compound in the form of colorless crystals.

NMR (CDCl₃): δ 0.07(6H,s,-Si(CH₃)₂), 0.88(9H,s, SiC(CH₃)₃), 1.13(3H,d,J=6.6Hz,CH₃CHOSi), 1.50 (3H,S,CH₃), 3.01-3.04(1H,m,C3-H), 4.18(1H,d,J=2.6Hz,C4-H), 4.17-4.25 (1H,m,CHOSi), 4.60-4.68(4H,m,CH₂CH=CH₂), 5.23-5.38(4H,m, CH₂CH=CH₂), 5.77-5.95(2H,m,CH₂CH=CH₂), 5.95(1H,brs,-NH).

### Example 11

### Synthesis of (3S,4S)-3-[(R)-1-(tert-butyldimethylsilyloxy)-ethyl]-4-[(RS)-1-allyloxycarbonyl-cyclohexan-2-on-1-yl]-2-a zetidinone

A solution of 2-allyloxycarbonylcyclohexanone (1.27 g, 6.98 mmole) in tetrahydrofuran (8 ml) was added dropwise to a suspension of sodium hydride (267 mg, 6.98 mmole) in tetrahydrofuran (30 ml) under an argon stream at 0°C, and the solution mixture was stirred for 10 min and admixed with cuprous bromide dimethylsulfide complex (1.44 g, 6.98 mmole), followed by stirring for 15 min at the same temperature, addition of a solution of (3R, 4S)-3-[(R) -1--(tert-butyldimethylsilyloxy)ethyl]-4-phenylthio-2--azetidinone (1.18 g, 3.49 mmole) in tetrahydrofuran (8 ml) and stirring for 40 min. The reaction solution was admixed with aqueous saturated ammonium chloride solution and extracted with ethyl acetate. The ethyl acetate layer was washed with aqueous saturated sodium chloride solution, dried over magnesium sulfate and concentrated under reduced pressure, and the residue was purified by column chromatography (50 g of silica gel; a 4/1 mixture of n-hexane/ethyl acetate) to give 340 mg (24 %) of the subject compound having the cyclohexanone ring in the R-configuration in the form of colorless crystals and 920 mg (64 %) of the subject compound having the cyclohexanone ring in the S-configuration in the form of a colorless oily substance.

R-configuration isomer: IR (KBr) cm⁻¹: 1718, 1767. NMR (CDCl₃): δ 0.06 and 0.07(total,6H,each s,Si(CH₃)₂), 0.87 (9H,s,SiC(CH₃)₃), 1.00(3H,d,J=5.9Hz,CH₃CHOSi), 1.51-1.90 (4H,m), 2.01-2.12(1H,m), 2.36-2.54(3H,m), 2.95-3.00(1H,m, C3-H), 4.17 -4.28(1H,m,CHOSi), 4.37(1H,d, J=2.0Hz, C4-H), 4.57-4.70(2H,m,CH₂CH=CH₂), 5.26-5.42 (2H,m, CH₂CH=CH₂), 5.67(1H,brs,-NH). 5.82-5.98(1H,m,CH₂CH=CH₂).

S-configuration isomer: IR (neat) cm⁻¹: 1711, 1766. NMR (CDCl₃): δ 0.06 and 0. 07 (total, 6H, each s,Si(CH₃)₂), 0.87 (9H,s,SiC(CH₃)₃), 1.20(3H,d,J=6.6Hz,CH₃CHOSi), 1.39-1.70 (3H,m), 1.75-1.88 (1H,m), 2.00-2.11(1H,m), 2.42-2.52(3H,m), 3.12-3.17 (1H,m,C3-H), 3.88(1H,d,J=2.0Hz, C4-H), 4.10-4.22 (1H,m, CHOSi), 4.63-4.68(2H,m,CH₂CH=CH₂), 5.25-5.37(2H,m, CH₂CH=CH₂), 5.785.78-5.92(1H,m,CH₂CH=CH₂), 5.93(1H,brs,-NH).

### Example 12

### Synthesis of (3S,4S)-3-[(R)-1-(tert-butyldimethylsilyloxy)-ethyl]-4-[(RS)-1-(hydroxycarbonyl) ethyl]-2-azetidinone

Triethylamine (60 µl, 0.430 mmole), formic acid (15 µl, 0.401 mmole), triphenylphosphine (3.8 mg, 0.014 mmole) and palladium acetate (1.6 mg, 0.007 mmole) were added to a solution of the compound (61 mg, 0.143 mmole) as obtained in Example 10 in 1,4-dioxane (1.5 ml), successively, under an argon stream, and the reaction mixture was stirred at 100°C for one and a half hours. The mixture was cooled and admixed with diethyl ether (7 ml) and 0.5N sodium hydroxide solution (7 ml), followed by stirring, and the aqueous layer was adjusted to pH 3 with 1N hydrochloric acid and extracted with ether (30 ml). The extract layer was dried over sodium sulfate and concentrated under reduced pressure to give 36 mg (83 %) of the subject compound as a mixture consisting of the α (R-isomer)/β (S-isomer) at a ratio of 3/1.

NMR (CDCl₃): δ 0.07 and 0.09(total,6H,s,Si(CH₃)₂), 0.88(9H,s,SiC(CH₃)₃) , 1.15-1.31 (6H,m,CH₃CHOSi and CH₂CHCO₂H), 2.55(0.75H,dd,J=7.3 and 9.9 Hz,CHCO₂H),
2.70-2.85(1H,m,CHCO₂H and C3-H), 3.02-3.06(0.25H,m,C3-H), 3.69 (0.75H,dd,J=2.0Hz and 9.9Hz,C4-H), 3.95(0.25H, dd, J=2.0Hz and 4.5Hz,C4-H), 4.12-4.25(1H,m,CHOSi), 6.45(0.25H, brs, -NH) , 6.79(0.75H,brs, NH).

### Example 13

### Synthesis of (3S,4S)-3-[(R)-1-(tert-butyldimethylsilyloxy)-ethyl]-4-[(RS)cyclohexan-2-on-1-yl]-2-azetidinone

Under an argon stream, a solution of triethylamine (87 µl, 0.625 mmole) and formic acid (19 µl, 0.50 mmole) in tetrahydrofuran (1 ml) was added dropwise to a solution of triphenylphosphine (6.6 mg, 0.025 mmole) and palladium acetate (2.8 mg, 0.0125 mmole) in tetrahydrofuran (1.5 ml), and then a solution of the S-isomer (102 mg, 0.25 mmole) of the compound as obtained in Example 11 in tetrahydrofuran (0.8 ml) was added dropwise to the resultant solution, followed by stirring at room temperature for 1 hour. The reaction mixture was admixed with aqueous saturated ammonium chloride solution (5 ml) and extracted with ethyl acetate (30 ml). The ethyl acetate layer was washed with aqueous saturated sodium chloride solution, dried over magnesium sulfate and concentrated under reduced pressure to give the subject compound as a mixture consisting of the α (R-isomer)/β (S-isomer) at a ratio of 2.5/1. Purification by column chromatography (5 g of silica gel; a 2/1 mixture of hexane/ethyl acetate) yielded 73 mg (90 %) of the subject compound as a mixture.

α-Form; IR (KBr) cm⁻¹: 1710, 1760. NMR (CDCl₃): δ 0.06 and 0.07 (total, 6H, each s,Si(CH₃)₂), 0.88(9H,s, SiC(CH₃)₃), 1.22(3H,d,J=5.9Hz,CH₃CHOSi), 1.32-1.48(1H.m), 1.61-1.76 (2H,m), 1.86-2.00(1H,m), 2.06-2.21(2H,m), 2.23-2.48(3H,m), 2.70(1H,dd,J=2.0 and 5.9 Hz,C3-H), 3.61 (1H,dd,J=2.0 and 9.9 Hz,C4-H), 4.07 -4.22(1H,m,CHOSi), 6.09(1H,brs,-NH).

β-Form; IR (KBr) cm⁻¹: 1706, 1755. NMR (CDCl₃): δ 0.06 and 0.07(total,6H,each s, Si(CH₃)₂), 0.87(9H,s, SiC(CH₃)₃), 1.23(3H,d,J=5.9Hz,CH₃CHOSi), 1.54-1.81(3H,m), 1.92-2.20 (3H,m), 2.26-2.48(2H,m), 2.51-2.61(1H,m), 2.87(1H,dd, J=2.0 and 4.7 Hz,C3-H), 4.06-4.12(1H,m,C4-H), 4.12-4.24 (1H,m,CHOSi), 5.72(1H,brs,NH).

### Example 14

### Synthesis of (3S,4S)-3-[(R)-1-(tert-butyldimethylsilyloxy)ethyl]-4-[1,1-di(allyloxycarbonyl)ethyl]-1-(p-nitrobenzyloxycarbonyl)-methyl-2-azetidinone

Potassium carbonate (323 mg, 1.61 mmole) was added to a solution of the compound (220 mg, 0.517 mmole) obtained in Example 10 and p-nitrobenzyl 1-iodoacetate (0.592 mmole) in N,N-dimethylformamide (5 ml) under an argon stream, followed by stirring at room temperature for 2 hours. The solution mixture was furthermore stirred at 50°C for 2 hours, cooled, admixed with water (10 ml) and extracted with diethyl ether (45 ml), and the ether layer was washed with aqueous saturated sodium chloride solution (15 ml), dried over magnesium sulfate and concentrated under reduced pressure. The residue was purified by column chromatography (10 g of silica gel, a 20/1 mixture of hexane/ethyl acetate) to give 148 mg (46 %) of the subject compound, with 120 mg (54 %) of the starting compound being recovered.

NMR (CDCl₃): 0.02 and and 0.06(total 6H,each s, Si(CH₃)₂), 0.86(9H,s, SiC(CH₃)₃), 1.20(3H,d,J=6.0Hz, CH₃CHOSi), 1.54(3H,s,CH₃), 3.05(1H,dd,J=2.0 and 5.9 Hz,C3-H), 3.90(1H,d,J=17.8Hz,C(H)HCO₂PNB), ), 4.08-4.19 (1H,m, CHOSi), 4.29(1H,d,J=17.8Hz,C(H)HCO₂PNB), 4.34(1H,d,J=2.0Hz, C4-H), 4.53-4.644.53-4.64(4H,m,CO₂CH₂CH=CH₂), ), 5.205.20-5.36(6H,m,CH₂Ar and CH₂CH=CH₂), 5.755.75-5.96(2H,m,CH₂CH=CH₂), 7.55(2H,d, J=9.2Hz,Ar), 8.22(2H,d,J=9.2Hz,Ar).

### Example 15

### Synthesis of (3S,4S)-3-[(R)-1-(tert-butyldimethylsilyloxy)-ethyl]-4-[(R)-1-allyloxycarbonyl-cyclohexan-2-on-1-yl]-1-(p -nitrobenzyl-oxycarbonyl) methyl-2-azetidinone

Potassium carbonate (101 mg, 0.733 mmole) was added to a solution of the compound (R-isomer) (100 mg, 0.244 mmole) obtained in Example 11 and p-nitrobenzyl 1-iodoacetate (0.293 mmole) in N,N-dimethylformamide (4 ml) under an argon stream, followed by stirring at room temperature for 12 hours. The solution mixture was furthermore stirred at 50°C for 2 hours, cooled, admixed with water (6 ml) and extracted with diethyl ether (30 ml), and the ether layer was washed with aqueous saturated sodium chloride solution (10 ml), dried over sodium sulfate and concentrated under reduced pressure. The residue was purified by column chromatography (10 g of silica gel, a 20/1 mixture of dichloromethane/ethyl /ethyl acetate) to give 39 mg (27 %) of the subject compound, with 48 mg (48 %) of the starting compound being recovered.

NMR (CDCl₃): δ 0.06 and 0.07(total 6H,each s, Si(CH₃)₂), 0.87(9H,s, SiC(CH₃)₃), 0.99(3H,d,J=6.5Hz, CH₃CHOSi), 1.52-1.71(2H,m), 1.83-1.95(2H,m), 2.21-2.33 (2H,m), 2.36-2.43(2H,m), 3.08-3.10(1H,m,C3-H), 4.09(1H,d, J=8.7Hz,C(H)HCO₂PNB), 4.18(1H,d,J=8.7Hz, C(H)HCO₂PNB), 4.18-4.25 (1H,m, CHOSi), 4.52(1H,d,J=2.0Hz, C4-H), 4.63(2H,ddd,J=4.5, 8.8 and 13.5 Hz,CO₂CH₂CH=CH₂), 5.13(1H,d,J=9.0Hz,C(H)Ar), 5.28(1H,d,J=9.0Hz, C(H)Ar), 5.29-5.38(2H,m,CH₂CH=CH₂), 5.83-5.95(1H,m,CH₂CH=CH₂), 7.54 (2H,d,J=9.2Hz,Ar), 8.22(2H,d,J=9.2Hz,Ar).

### Example 16

### Synthesis of (3S,4S)-3-[(R)-1-(tert-butyldimethylsilyloxy)-ethyl]-4-[(RS)-1-hydroxycarbonyl)ethyl]-1-(p-nitrobenzyloxycarbonyl) methyl-2-azetidinone

Triethylamine (32 µl, 0.233 mmole), formic acid (7.3 µl, 0.194 mmole), triphenylphosphine (2.0 mg, 0.0077 mmole) and palladium acetate (1.6 mg, 0.0038 mmole) were added to a solution of the compound (48 mg, 0.0775 mmole) as obtained in Example 14 in 1,4-dioxane (1.5 ml), successively, under an argon stream, and the reaction mixture was stirred at 100°C for one hour. The mixture was cooled, admixed with potassium hydrogen sulfate (5 ml) and extracted with ethyl acetate (25 ml). The ethyl acetate layer was washed with aqueous saturated sodium chloride solution, dried over sodium sulfate and concentrated under reduced pressure to give 45 mg (quant.) of the subject compound as a mixture consisting of the a (R-isomer)/β (S-isomer) at a ratio of 1/1.7.

NMR (CDCl₃): δ 0.02-0. 08 ( 6H, m, SiC (CH₃) ₂) , 0.85 and 0.86(total,9H,each s, Si(CH₃)₃), 1.19-1.28(6H,m,CH₃CHOSi and CH₃CHCO₂H) , 2.71(0.37H,dd,J=7.1 and 7.4 HZ, CHCO₂H), 2.83-2.92(1H,m,CHCO₂H and C3-H), 3.05(0.63H,dd,J=2.0 and 6.0 Hz, C3-H), 3.90-4.32 (4H,m,CHOSi,CH₂CO₂ and C4-H), 5.08-5.33 (2H,m,CO₂CH₂), 7.45-7.70(4H,m,Ar).

### Example 17

### synthesis of (3S,4S)-3-[(R)-1-(tert-butyldimethylsilyloxy)-ethyl]-4-[(RS)-1-cyclohexan-2-on-1-yl]-1-(p-nitrobenzyloxyc arbonyl)methyl-2-azetidinone

Triethylamine (13.6 µl, 0.0972 mmole), formic acid (2.9 µl, 0.078 mmole), triphenylphosphine (1.0 mg, 0.0038 mmole) and palladium acetate (0.4 mg, 0.0019 mmole) were added to a solution of the compound (20 mg, 0.0331 mmole) as obtained in Example 15 in tetrahydrofuran (1 ml) , successively, under an argon stream, and the reaction mixture was stirred at room temperature for one and a half hours. The mixture was admixed with aqueous saturated sodium chloride solution and extracted with ethyl acetate. The ethyl acetate layer was dried over sodium sulfate and concentrated under reduced pressure to give the subject compound as a mixture consisting of the α (R-isomer)/β (S-isomer) at a ratio of 1/1.3. Purification of the residue by column chromatography (5 g of silica gel; a 5/1 mixture of hexane/ethyl acetate) yielded 4 mg (23 %) of the α-form and 7 mg (41 %) of the β-form.

α-Form (R-isomer): NMR (CDCl₃): δ 0.03 and 0.06 (total 6H,each s,Si(CH₃)₂), 0.86(9H,s,SiC(CH₃)₃), 1.22(3H,d, J=5.9Hz,CH₃CHOSi), 1.23-1.44(1H,m), 1.55-1.76(2H,m), 1.80-1.97 (1H,m), 2.04-2.18 (2H,m), 2.18-2.31 (2H,m), 2.61-2.75(1H,m,C4CHC=O), 2.80(1H,dd,J=2.0 and 5.3 Hz,C3-H), 3.88(1H,dd,J=2.0 and 9.9 Hz,C4-H), 4.09(1H,d, J=17.8Hz, CH(H)CO₂PNB), 4.10-4.20(1H,m,CHOSi), 4.23(1H,d, J=17.8Hz, CH(H)CO₂PNB), 5.18(2H,q,J=13.2Hz,CH₂Ar), 7.50(2H,d,J=9.2Hz, Ar), 8.22(2H,d,J=9.2Hz,Ar).

β-Form (S-isomer): NMR (CDCl₃): δ 0.01 and 0.05(total 6H,each s,Si(CH₃)₂), 0.84(9H,s,SiC(CH₃)₃), 1.24(3H,d, J=5.9Hz,CH₃CHOSi), 1. 56-1.89 (3H,m) , 1.95-2.21 (3H,m) , 2.23-2.39 (2H,m), 2.56-2.66(1H,m,C4CHC=O), 2.95(1H,dd,J=2.6 and 7.2 Hz,C3-H), 3.90(1H,d,J=17.8Hz,C(H)HCO₂PNB), 4.18(1H,d, J=17.8Hz,C(H)HCO₂PNB), 4.09-4.21(1H,m,CHOSi), 4.28-4.31(1H,m,C4-H), 5.26(2H,d,J=9.2Hz,CH₂Ar), 7.55(2H,d, J=9.2Hz,Ar), 8.23(2H,d,J=9.2Hz,Ar).

As has been described above, the present invention can provide a process of converting the 4-position substituent of the azetidinone derivatives of the general formula [1] in one step to produce the derivatives of the general formula [3], and also can allow the derivatives [3] and [4] to undergo a decarboxylation reaction to give the azetidinone derivatives of the general formula [5], thereby enabling the azetidinone derivatives of the general formula [5] to be synthesized easily in a decreased number of steps as compared with the conventionally known procedures.

## Claims

1. A process for synthesizing a 4-substituted azetidinone derivative represented by the general formula [3]: 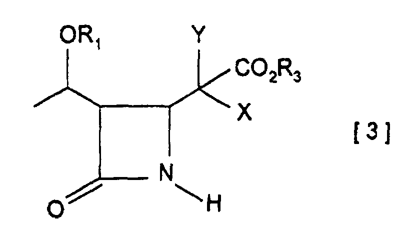 (wherein OR₁ is a protected hydroxyl group, CO₂R₃ is an esterified carboxyl group, and X and Y are the same or different and represent individually a substituted or unsubstituted alkyl group, substituted or unsubstituted alkenyl group, substituted or unsubstituted aralkyl group, substituted or unsubstituted aryl group, substituted or unsubstituted alkylthio group, substituted or unsubstituted alkenylthio group, substituted or unsubstituted aralkylthio group, substituted or unsubstituted arylthio group, substituted or unsubstituted alkyloxy group, substituted or unsubstituted alkenyloxy group, substituted or unsubstituted aralkyloxy group, substituted or unsubstituted aryloxy group, substituted or unsubstituted silyloxy group, substituted or unsubstituted heterocyclic group, substituted or unsubstituted heterocyclic thio group, substituted or unsubstituted heterocyclic oxy group, substituted or unsubstituted acyl group, substituted or unsubstituted ester group, substituted or unsubstituted thioester group, substituted or unsubstituted amide group, substituted or unsubstituted amino group, hydrogen atom or halogen atom, or with the carbon atom to which they are attached form a substituted or unsubstituted cycloalkan-2-on-1-yl group),
**characterized in that** said process comprises
(a) reacting an azetidinone derivative represented by the general formula [1]: 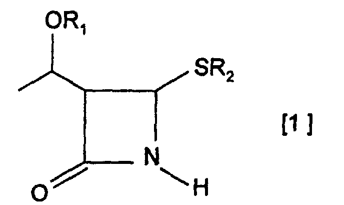 (wherein OR₁ is as defined above; R₂ is an alkyl, alkenyl, C₆ - C₁₀ aryl or aromatic heterocyclic group, which group may have substituents selected from halogen, C₁ - C₆ alkyl, monocyclic or polycyclic alkyl, C₁ - C₆ alkoxy, carboxyl, amino, nitro, cyano, hydroxy, C₆ - C₁₀ aryl and C₇ - C₂₄ aralkyl)
with an ester compound represented by the general formula [2]: 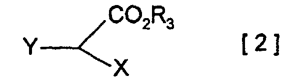 (wherein CO₂R₃, X and Y are as defined above) in the presence of zinc and a copper compound selected from copper oxides, copper halides, salts of copper with organic carboxylic acids, salts of copper with mineral acids, and complexes of cuprous halides, or
(b) treating an ester compound represented by the general formula [2] with a metal base to form the corresponding metal enolate, followed by reaction with an azetidinone derivative represented by the general formula [1] in the presence of a copper compound selected from copper oxides, copper halides, salts of copper with organic carboxylic acids, salts of copper with mineral acids, and complexes of cuprous halides.

2. A process as claimed in Claim 1, wherein an azetidinone derivative represented by the general formula [1] is reacted with an ester compound represented by the general formula [2].

3. A process as claimed in Claim 1, wherein an ester compound represented by the general formula [2] is treated with a metal base to form the corresponding metal enolate, followed by reaction with an azetidinone derivative represented by the general formula [1] in the presence of a copper compound.

4. A process as claimed in any one of the preceding claims, wherein CO₂R₃ is an esterified carboxyl group selected from tri-substituted silyl esters, tri-substituted silyl C₁- C₆-alkyl esters, aromatic heterocyclic esters, C₁ - C₆ alkyl esters, C₁ - C₆ alkanoyloxy C₁ - C₆ alkyl esters, C₁ - C₆ alkanesulfonyl C₁ - C₆ alkyl esters, mono or di or tri halo C₁ - C₆ alkyl esters, C₁ - C₆ alkoxycarbonyloxy C₁ - C₆ alkyl esters, phthalidylidene C₁ - C₆ alkyl esters, (5- C₁ - C₆-alkyl-2-oxo-1,3- dioxolane-4-yl) ( C₁- C₆) alkyl esters, C₁ - C₆ alkenyl esters, C₁ - C₆ alkynyl esters, aryl C₁ - C₆ alkyl esters, aryl esters, and phthalidyl esters, which may optionally be substituted

5. A process as claimed in any one of the preceding claims, wherein any ester or thioester group represented by X or Y is selected from alkyloxycarbonyl, alkenyloxycarbonyl, aralkyloxycarbonyl, aryloxycarbonyl, alkylthiocarbonyl, alkenylthiocarbonyl, aralkylthiocarbonyl, arylthiocarbonyl,

6. A process as claimed in any one of the preceding claims, wherein any substituents on R₃ are selected from halogen, C₁ - C₆ alkyl, monocyclic or polycyclic alkyl, C₁ - C₆ alkoxy, carboxyl, amino, nitro, cyano, hydroxy, C₆ - C₁₀ aryl, C₇ - C₂₄ aralkyl, heterocyclic, acyl, alkyloxycarbonyl, alkenyloxycarbonyl, aralkyloxycarbonyl, and aryloxycarbonyl.

7. A process as claimed in any one of the preceding claims, wherein any substituents on X and Y are selected from halogen, carboxyl group, formyl group, nitro, cyano, hydroxyl, amino group, C₁ - C₆ alkyl, monocyclic and polycyclic alkyl, C₁ - C₆ alkenyl, C₆ - C₁₀ aryl, C₇ - C₂₄ aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, carbamoyl; carbamoyloxy, imino-C₁ - C₆-alkyl, imino- C₁ - C₆-alkylamino, acyloxy, silyloxy, heterocyclic, heterocyclic-thio, heterocyclic-oxy, acyl carboxyl, alkyloxycarbonyl, alkenyloxycarbonyl, aralkyloxycarbonyl, aryloxycarbonyl, thiocarboxyl, alkylthiocarbonyl, alkenylthiocarbonyl, aralkylthiocarbonyl, arylthiocarbonyl, and substituted and unsubstituted amide.

8. A process as claimed in any one of the preceding claims, wherein the ester compound represented by the general formula [2] is a halogenated acetic acid ester, a malonic acid ester, an 2-alkylmalonic acid ester, a 2-halogenated malonic acid ester, an 2-alkyl-acylacetic acid ester or an cycloalkan-2-on-1-carboxylic acid ester.

9. A process as claimed in Claim 8, wherein the ester compound represented by the general formula [2] is a bromoacetic acid ester, a malonic acid ester, a 2-methylmalonic acid ester, a 2-fluoromalonic acid ester, a 2-methylacetoacetic acid ester or a cyclohexan-2-on-1-carboxylic acid ester.

10. A process as claimed in Claim 2, wherein (3S,4S)-3-[(R)-1-(tert-butyldimethylsilyloxy)- ethyl]-4-ethoxycarbonylmethyl-2-azetidinone is prepared from ethyl bromoacetate and (3S,4R)-3-[(R)-1-(tert-butyldimethylsilyloxy)ethyl]-4-phenylthio-2-azetidinone.

11. A process as claimed in Claim 3, wherein (3S,4S)-3-[(R)-1-(tert-butyldimethylsilyloxy)- ethyl]-4-di(methoxycarbonyl)-methyl-2-azetidinone is prepared from dimethyl malonate and (3S,4R)-3-[(R)-1-(tert-butyldimethylsilyloxy) -ethyl] -4-phenylthio-2-azetidinone.

12. A process as claimed in Claim 3, wherein (3S,4S)-3-[(R)-l-(tert-butyldimethylsilyloxy)- ethyl]-4-[1,1-di (ethoxycarbonyl)ethyl-2-azetidinone is prepared from diethyl 2-methylmalonate and (3S,4R)-3-[(R)-1- (tert-butyldimethylsilyloxy) ethyl] -4-phenylthio-2-azetidinone.

13. A process as claimed in Claim 3, wherein (3S,4S)-3-[(R)-1-(tert-butyldimethylsilyloxy)- ethyl]-4-di(ethoxycarbonyl)fluoromethyl-2-azetidinone is prepared from diethyl 2-fluoromalonate and (3S,4R)-3-[(R)-1- (tert-butyldimethylsilyloxy) ethyl] -4-phenylthio-2-azetidinone.

14. A process as claimed in Claim 3, wherein (3S,4S)-3-[(R)-1-(tert-butyldimethylsilyloxy)- ethyl]-4-(1-acetyl-1-ethoxycarbonylethyl)-2-azetidinone is prepared from ethyl 2-methylacetoacetate and (3S,4R)-3-[(R)-1-(tert-butyldimethylsilyloxy) ethyl]-4-phenylthio-2-azetidinone.

15. A process as claimed in Claim 3, wherein (3S,4S)-3-[ (R) -1-(tert-butyldimethylsilyloxy)- ethyl]-4-(1-acetyl-1-allyloxycarbonylethyl)-2-azetidinone is prepared from allyl 2-methylacetoacetate and (35,4R)-3-[(R)-1-(tert-butyldimethyl- silyloxy)ethyl]-4-phenylthio-2-azetidinone.

16. A process as claimed in Claim 3, wherein (3S,4S)-3-[(R)-1-(tert-butyldimethylsilyloxy)- ethyl]-4 -(1-acetyl-1-benzyloxycarbonylethyl)-2-azetidinone is prepared from benzyl 2-methylacetoacetate and (3S,4R)-3-[(R)-1-(tert-butyldimethyl- silyloxy)ethyl]-4-phenylthio-2-azetidinone.

17. A process as claimed in Claim 3, wherein (3S,4S)-3-[(R)-1-(tert-butyldimethylsilyloxy)ethyl] -4-[1,1-di(allyloxycarbonyl)ethyl]-2-azetidinone is prepared from diallyl 2-methylmalonate and (3R,4S)-3-[(R)-1-(tert-butyldimethylsilyloxy)ethyl]-4-phenylthio-2-azetidinone.

18. A process as claimed in Claim 3, wherein (3S,4S)-3-((R)-1-(tert-butyldimethylsilyloxy)ethyl]-4-[(RS )-1-allyloxycarbonyl-cyclohexan-2-on-1-yl] -2-azetidinone is prepared from 2-allyloxycarbonylcyclohexanone and (3R,4S)-3-[(R)-1-(tert-butyldimethylsilyloxy)ethyl]-4-phenylthio-2-azetidinone.

19. A process as claimed in any one of the preceding claims, wherein the copper compound is a cuprous bromide dimethylsulfide complex.

## Patentansprüche

1. Verfahren zur Synthese eines 4 - substituierten Azetidinonderivats, das durch die allgemeine Formel [3] dargestellt wird: 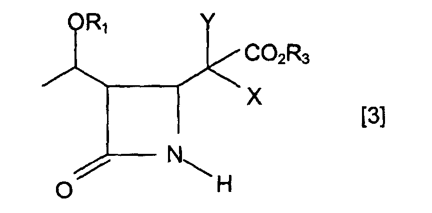 (worin OR₁eine geschützte Hydroxylgruppe und CO₂R₃ eine veresterte Carboxylgruppe bedeuten, wobei X und Y gleich oder verschieden sind und unabhängig voneinander einen substituierten oder unsubstituierten Alkylrest, einen substituierten oder unsubstituierten Alkenylrest, einen substituierten oder unsubstituierten Aralkylrest, einen substituierten oder unsubstituierten Arylrest, einen substituierten oder unsubstituierten Alkylthiorest, einen substituierten oder unsubstituierten Alkenylthiorest, einen substituierten oder unsubstituierten Aralkylthiorest, einen substituierten oder unsubstituierten Arylthiorest, einen substituierten oder unsubstituierten Alkyloxyrest, einen substituierten oder unsubstituierten Alkenyloxyrest, einen substituierten oder unsubstituierten Aralkyloxyrest, einen substituierten oder unsubstituierten Aryloxyrest, einen substituierten oder unsubstituierten Silyloxyrest, einen substituierten oder unsubstituierten heterocyclischen Rest, eine substituierte oder unsubstituierte heterocyclische Thiogruppe, eine substituierte oder unsubstituierte heterocyclische Oxygruppe, einen substituierten oder unsubstituierten Acylrest, eine substituierte oder unsubstituierte Estergruppe, eine substituierte oder unsubstituierte Thioestergruppe, eine substituierte oder unsubstituierte Amidgruppe, eine substituierte oder unsubstituierte Aminogruppe, ein Wasserstoffatom oder Halogenatom bedeuten, oder mit dem Kohlenstoffatom, mit dem sie verknüpft sind, einen substituierten oder unsubstituierten Cycloalkan - 2 - on - 1 - ylrest bilden),
**dadurch gekennzeichnet, dass** das genannte Verfahren folgende Maßnahmen umfasst:
(a) die Reaktion eines durch die allgemeine Formel [1] dargestellten Azetidinonderivats: 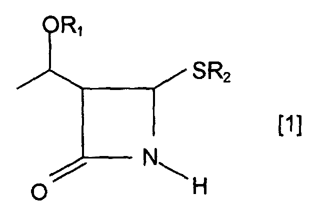 (worin OR₁ die oben stehende Bedeutung aufweist und R₂ einen Alkyl-, Alkenyl-, C₆ - C₁₀ - - Arylrest oder einen aromatischen heterocyclischen Rest bedeutet, wobei dieser Rest Substituenten aufweisen kann, die aus Halogen, C₁ - C₆ - Alkyl, monocyclischem oder polycyclischem Alkyl, C₁ - - C₆ - Alkoxy, Carboxyl, Amino, Nitro, Cyano, Hydroxy, C₆ - C₁₀ - Aryl und C₇ - C₂₄- Aralkyl ausgewählt sind)
zusammen mit einer durch die allgemeine Formel [2] symbolisierten Esterverbindung: 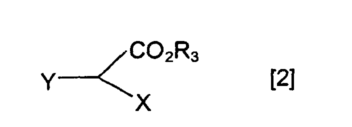 (worin CO₂R₃, X und Y die oben stehenden Bedeutungen aufweisen)
in Anwesenheit von Zink und einer Kupferverbindung, die aus Kupferoxiden, Kupferhalogenverbindungen sowie Salzen aus Kupfer mit organischen Carbonsäuren, Salzen aus Kupfer mit Mineralsäuren und Komplexen aus Kupfer(I)-Halogenverbindungen ausgewählt ist, oder
(b) Behandlung einer durch die allgemeine Formel [2] symbolisierten Esterverbindung mit einer metallischen basischen Verbindung zur Darstellung des entsprechenden Metallenolats, gefolgt durch die Reaktion mit einem Azetidinonderivat, das durch die allgemeine Formel [1] symbolisiert wird, in Anwesenheit einer Kupferverbindung, die aus Kupferoxiden, Kupferhalogenverbindungen sowie Salzen aus Kupfer mit organischen Carbonsäuren, Salzen aus Kupfer mit Mineralsäuren und Komplexen aus Kupfer(I)-Halogenverbindungen ausgewählt ist.

2. Verfahren nach Anspruch 1, worin ein Azetidinonderivat, das durch die allgemeine Formel [1] symbolisiert wird, zusammen mit einer durch die allgemeine Formel [2] symbolisierten Esterverbindung zur Reaktion gebracht wird.

3. Verfahren nach Anspruch 1, worin eine durch die allgemeine Formel [2] symbolisierte Esterverbindung mit einer metallischen basischen Verbindung zur Darstellung des entsprechenden Metallenolats behandelt wird, gefolgt durch die Reaktion mit einem Azetidinonderivat, das durch die allgemeine Formel [1] symbolisiert wird, in Anwesenheit einer Kupferverbindung.

4. Verfahren nach einem der vorstehenden Ansprüche, worin CO₂R₃ eine veresterte Carboxylgruppe bedeutet, die ausgewählt ist aus dreifach substituierten Silylestern, dreifach substituierten Silyl - C₁ - C₆ - Alkylestern, aromatischen heterocyclischen Estern, C₁ - C₆ - Alkylestern, C₁ - C₆ - Alkanoytoxy - C₁ - C₆ - Alkylestern, C₁ - C₆ - Alkansulfonyl - C₁ - C₆ - Alkylestern, Mono- oder Dioder Trihalogen - C₁ - C₆ - Alkylestern, C₁ - C₆ - Alkoxycarbonyloxy - C₁ - C₆ - Alkylestern, Phthalidyliden - C₁ - C₆ - Alkylestern, (5 - C₁ - C₆ - Alkyl - 2 - oxo - 1,3 - dioxolan - 4 - yl) - C₁ - C₆ - - alkylestern, C₁ - C₆ - Alkenylestern, C₁ - C₆ - Alkinylestern, Aryl - C₁ - C₆ - Alkylestern, Arylestern sowie Phthalidylestern, die je nach Wunsch substituiert sein können.

5. Verfahren nach einem der vorstehenden Ansprüche, worin eine beliebige Ester- oder Thioestergruppe, die durch X oder Y symbolisiert wird, aus Alkyloxycarbonyl, Alkenyloxycarbonyl, Aralkyloxycarbonyl, Aryloxycarbonyl, Alkylthiocarbonyl, Alkenylthiocarbonyl, Aralkylthiocarbonyl oder Arylthiocarbonyl ausgewählt wird.

6. Verfahren nach einem der vorstehenden Ansprüche, worin beliebige Substituenten im Rest R₃ aus Halogen, C₁ - C₆ - Alkyl, monocyclischem oder polycyclischem Alkyl, C₁ - C₆ - Alkoxy, Carboxyl, Amino, Nitro, Cyano, Hydroxy, C₆ - C₁₀ - Aryl, C₇- C₂₄- Aralkyl, Heterocyclen, Acyl, Alkyloxycarbonyl, Alkenyloxycarbonyl, Aralkyloxycarbonyl und Aryloxycarbonyl ausgewählt sind.

7. Verfahren nach einem der vorstehenden Ansprüche, worin beliebige Substituenten an X and Y ausgewählt sind aus Halogen, einer Carboxylgruppe, einer Formylgruppe, Nitro, Cyano, Hydroxyl, einer Aminogruppe, C₁ - C₆ - Alkyl, monocyclischem und polycyclischem Alkyl, C₁ - C₆ - Alkenyl, C₆ - C₁₀ - - Aryl, C₇ - C₂₄ - Aralkyl, Alkylthio, Alkenylthio, Aralkylthio, Arylthio, Alkyloxy, Alkenyloxy, Aralkyloxy, Aryloxy, Alkylsulfinyl, Alkylsulfonyl, Aralkylsulfinyl, Aralkylsulfonyl, Arylsulfinyl, Arylsulfonyl, Carbamoyl; Carbamoyloxy, Imino - C₁ - C₆ - Alkyl, Imino - C₁ - C₆ - Alkylamino, Acyloxy, Silyloxy, Heterocyclen, Heterocyclus-thio, Heterocyclus-oxy, Acylcarboxyl, Alkyloxycarbonyl, Alkenyloxycarbonyl, Aralkyloxycarbonyl, Aryloxycarbonyl, Thiocarboxyl, Alkylthiocarbonyl, Alkenylthiocarbonyl, Aralkylthiocarbonyl, Arylthiocarbonyl sowie substituiertem und unsubstituiertem Amid.

8. Verfahren nach einem der vorstehenden Ansprüche, worin die durch die allgemeine Formel [2] symbolisierte Esterverbindung einen halogenierten Essigsäureester, einen Malonsäureester, einen 2-Al-kylmalonsäureester, einen 2-halogenierten Malonsäureester, einen 2 - Alkylacylessigsäureester oder einen Cycloalkan - 2 - on - 1 - carbonsäureester darstellt.

9. Verfahren nach Anspruch 8, worin die durch die allgemeine Formel [2] symbolisierte Esterverbindung einen Bromessigsäureester, einen Malonsäureester, einen 2 - Methylmalonsäureester, einen 2 - Fluormalonsäureester, einen 2 - Methylacetoessigsäureester oder einen Cyclohexan - 2 - on - 1 - carbonsäureester darstellt.

10. Verfahren nach Anspruch 2, worin (35, 4S) - 3 - [(R) - 1 - *(tert. -* Butyldimethylsilyloxy)-- ethyl] - 4 - ethoxycarbonylmethyl - 2 - azetidinon aus Ethylbromacetat und (3S, 4R) - 3 - [(R) - *- 1 - (tert. -* Butyldimethylsilyloxy) - ethyl] - 4 - phenylthio - 2 - azetidinon hergestellt wird.

11. Verfahren nach Anspruch 3, worin (35, 4S) - 3 - [(R) - 1 - *(tert. -* Butyldlmethylsilyloxy)-- ethyl] - 4 - di-(methoxycarbonyl)- methyl - 2 - azetidinon aus Dimethylmalonat und (3S, 4R) - 3 - - [(R) - 1 - (*tert.* -Butyldimethylsilyloxy) - ethyl] - 4 - phenylthio - 2 - azetidinon hergestellt wird.

12. Verfahren nach Anspruch 3, worin (3S, 45) - 3 - [(R) - 1 - (*tert.* - Butyldimethylsilyloxy)-- ethyl] - 4 -[1,1 - di-(ethoxycarbonyl)- ethyl - 2 - azetidinon aus Diethyl - 2 - methylmalonat und (3S, 4R) - 3 - [(R) - 1 - (*tert. -* Butyldimethylsilyloxy) - ethyl] - 4 - phenylthio - 2 - azetidinon hergestellt wird.

13. Verfahren nach Anspruch 3, worin (35, 45) - 3 - [(R) - 1- *(tert. -* Butyldlmethytsltyloxy)-- ethyl] - 4 - di-(ethoxycarbonyl)- fluoromethyl - 2 - azetidinon aus Diethyl - 2 - Fluormalonat und (35, 4R) - 3 - [(R) - 1 - *(tert.-* Butyldimethylsilyloxy)- ethyl] - 4 - phenylthio - 2 - azetidinon hergestellt wird.

14. Verfahren nach Anspruch 3, worin (35, 45) - 3 - [(R) - 1 - *(tert. -* Butyldimethylsilyloxy)-- ethyl] - 4 - (1 - acetyl - 1 - ethoxycarbonylethyl) - 2 - azetidinon aus Ethyl - 2 - Methylacetoacetat und (35, 4R) - 3 - [(R) - 1 - *(tert. -* Butyldimethylsilyloxy)- ethyl] - 4 - phenylthio - 2 - azetidinon hergestellt wird.

15. Verfahren nach Anspruch 3, worin (35, 45) - 3 - [(R) - 1 - (*tert.*- Butyldimethylsilyloxy)-- ethyl] - 4 - (1 - acetyl - 1 -allyloxycarbonylethyl)- 2 - azetidinon aus Allyl - 2 - methylacetoacetat und (35, 4R) - 3 - [(R) - 1 - *(tert. -* Butyldimethylsilyloxy)- ethyl] - 4 - phenylthio - 2 - azetidinon hergestellt wird.

16. Verfahren nach Anspruch 3, worin (3S, 45) - 3 - [(R) - 1 - *(tert.-* Butyldimethylsilyloxy)-- ethyl] - 4 - (1-acetyl - 1 - benzyloxycarbonylethyl)- 2 - azetidinon aus Benzyl - 2 - methylacetoacetat und (35, 4R) - 3 - [(R) - 1 - *(tert. -* Butyldimethylsilyloxy)- ethyl] - 4 - phenylthio - 2 - azetidinon hergestellt wird.

17. Verfahren nach Anspruch 3, worin (35, 45) - 3 - [(R) - 1 - *(tert. -* Butyldimethylsilyloxy)-- ethyl] - 4 -[1, 1 - di-(allyloxycarbonyl)- ethyl] - 2 - Azetidinon aus Diallyl - 2 - methylmalonat und (3R, 45) - 3 - [(R) - 1 - *(tert. -* Butyldimethylsilyloxy)- ethyl] - 4 - phenylthio - 2 - azetidinon hergestellt wird.

18. Verfahren nach Anspruch 3, worin (35, 45) - 3 - [(R) - 1 - *(tert. -* Butyldimethylsilyloxy)-- ethyl] - 4 - [(RS) - 1 - allyloxycarbonyl - cyclohexan - 2 - on - 1 - yl] - 2 - azetidinon aus 2 - - Allyloxycarbonylcyclohexanon und (3R, 45) - 3 - [(R) - 1 - (*tert.* - Butyldimethylsilyloxy)- ethyl] - - 4- phenylthio - 2 - azetidinon hergestellt wird.

19. Verfahren nach einem der vorstehenden Ansprüche, worin die Kupferverbindung in Form eines Kupfer(I) - Bromid - Dimethylsulfid - Komplexes vorliegt.

## Revendications

1. Procédé pour synthétiser un dérivé azétidinone 4-substitué représenté par la formule générale [3] : 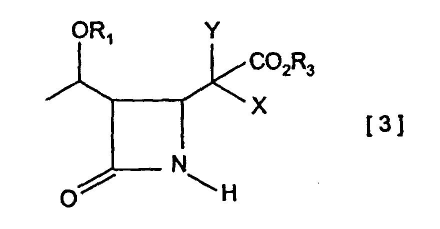 (dans laquelle OR₁ est un radical hydroxyle protégé, CO₂R₃ est un radical carboxyle estérifié, et X et Y sont identiques ou différents et représentent individuellement un radical alkyle substitué ou non substitué, un radical alcényle substitué ou non substitué, un radical aralkyle substitué ou non substitué, un radical aryle substitué ou non substitué, un radical alkylthio substitué ou non substitué, un radical alcénylthio substitué ou non substitué, un radical aralkylthio substitué ou non substitué, un radical arylthio substitué ou non substitué, un radical alkyloxy substitué ou non substitué, un radical alcényloxy substitué ou non substitué, un radical aralkyloxy substitué ou non substitué, un radical aryloxy substitué ou non substitué, un radical silyloxy substitué ou non substitué, un radical hétérocyclique substitué ou non substitué, un radical thio hétérocyclique substitué ou non substitué, un radical oxy hétérocyclique substitué ou non substitué, un radical acyle substitué ou non substitué, un radical ester substitué ou non substitué, un radical thioester substitué ou non substitué, un radical amide substitué ou non substitué, un radical amino substitué ou non substitué, un atome d'hydrogène ou un atome d'halogène, ou avec l'atome de carbone auquel ils sont fixés, forment un radical cycloalcan-2-on-1-yle substitué ou non substitué),
**caractérisé en ce que** ledit procédé consiste à :
(a) faire réagir un dérivé azétidinone représenté par la formule générale [1] : 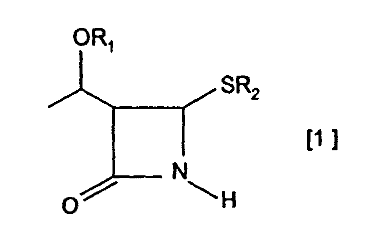 (dans laquelle OR₁ est tel que défini ci-dessus ; R₂ est un radical alkyle, alcényle, aryle en C₆ à C₁₀ ou hétérocyclique aromatique, radical qui peut avoir des substituants choisis parmi un atome d'halogène, les radicaux alkyle en C₁ à C₆, alkyle monocyclique ou polycyclique, alcoxy en C₁ à C₆, carboxyle, amino, nitro, cyano, hydroxy, aryle en C₆ à C₁₀ et aralkyle en ^{C}₇ à C₂₄)
avec un composé ester représenté par la formule générale [2] : 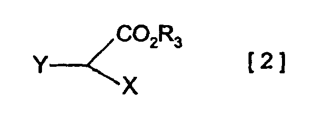 (dans laquelle CO₂R₃, X et Y sont tels que définis ci-dessus)
en présence de zinc ou d'un composé du cuivre choisi parmi les oxydes du cuivre, les halogénures du cuivre, les sels du cuivre ayant des acides carboxyliques organiques, les sels du cuivre ayant des acides minéraux et les complexes d'halogénures cuivreux ; ou
(b) traiter un composé ester représenté par la formule générale [2] avec une base métallique afin de former l'énolate de métal correspondant, suivi par la réaction avec un dérivé azétidinone représenté par la formule générale [1] en présence d'un composé du cuivre choisi parmi les oxydes du cuivre, les halogénures du cuivre, les sels du cuivre ayant des acides organiques carboxyliques, les sels du cuivre avec les acides minéraux et les complexes d'halogénures cuivreux.

2. Procédé selon la revendication 1, dans lequel un dérivé azétidinone représenté par la formule générale [1] est mis à réagir avec un composé ester représenté par la formule générale [2].

3. Procédé selon la revendication 1, dans lequel un composé ester représenté par la formule générale [2] est traité avec une base métallique afin de former l'énolate de métal correspondant, suivi par la réaction avec un dérivé azétidinone représenté par la formule générale [1] en présence d'un composé du cuivre.

4. Procédé selon l'une quelconque des revendications précédentes, dans lequel CO₂R₃ est un radical carboxyle estérifié choisi parmi les esters silyliques tri-substitués, les esters (alkyle en C₁ à C₆)silyliques tri-substitués, les esters hétérocycliques aromatiques, les esters d'alkyle en C₁ à C₆, les esters (alcanoyloxy en C₁ à C₆)-(alkyle en C₁ à C₆), les esters alcane(en C₁ à C₆)sulfonyle-(alkyle en C₁ à C₆), les esters mono, di ou tri-halogéno(alkyle en C₁ à C₆), les esters alcoxy(en C₁ à C₆)carbonyloxy-(alkyle en C₁ à C₆), les esters (alkyle en C₁ à C₆) phtalidylidène, les esters (5-(alkyle en C₁ à C₆)-2-oxo-1,3-dioxolane-4-yl)-(alkyle en C₁ à C₆), les esters (alcényle en C₁ à C₆), les esters (alcynyle en C₁ à C₆), les esters aryl(alkyle en C₁ à C₆), les esters aryliques, ainsi que les esters phtalidyliques, qui peuvent être éventuellement substitués.

5. Procédé selon l'une quelconque des revendications précédentes, dans lequel tout radical ester ou thioester représenté par X ou Y est choisi parmi les radicaux alkyloxycarbonyle, alcényloxycarbonyle, aralkyloxycarbonyle, aryloxycarbonyle, alkylthiocarbonyle, alcénylthiocarbonyle, aralkylthiocarbonyle, arylthiocarbonyle.

6. Procédé selon l'une quelconque des revendications précédentes, dans lequel tous substituants sur R₃ sont choisis parmi un atome d'halogène, les radicaux alkyle en C₁ à C₆, alkyle monocyclique ou polycyclique, alcoxy en C₁ à C₆, carboxyle, amino, nitro, cyano, hydroxy, aryle en C₆ à C₁₀, aralyle en C₇ à C₂₄, hétérocycliques, acyle, alkyloxycarbonyle, alcényloxycarbonyle, aralkyloxycarbonyle et aryloxycarbonyle.

7. Procédé selon l'une quelconque des revendications précédentes, dans lequel tous substituants sur X et Y sont choisis parmi un atome d'halogène, les radicaux carboxyle, formyle, nitro, cyano, hydroxyle, amino, alkyle en C₁ à C₆, alkyle monocyclique et polycyclique, alcényle en C₁ à C₆, aryle en C₆ à C₁₀, aralkyle en C₇ à C₂₄, alkylthio, alcénylthio, aralkylthio, arylthio, alkyloxy, alcényloxy, aralkyloxy, aryloxy, alkylsulfinyle, alkylsulfonyle, aralkylsulfinyle, aralkylsulfonyle, arylsulfinyle, arylsulfonyle, carbamoyle ; carbamoyloxy, imino-(alkyle en C₁ à C₆), imino-(alkyle en C₁ à C₆)-amino, acyloxy, silyloxy, hétérocyclique, hétérocyclique-thio, hétérocyclique-oxy, acylcarboxyle, alkyloxycarbonyle, alcényloxycarbonyle, aralkyloxycarbonyle, aryloxycarbonyle, thiocarboxyle, alkylthiocarbonyle, alcénylthiocarbonyle, aralkylthiocarbonyle, arylthiocarbonyle et l'amide substitué et non substitué.

8. Procédé selon l'une quelconque des revendications précédentes, dans lequel le composé ester représenté par la formule générale [2] est un ester d'acide acétique halogéné, un ester d'acide malonique, un ester d'acide 2-alkyl-malonique, un ester d'acide malonique 2-halogéné, un ester d'acide 2-alkyl-acylacétique ou un ester d'acide cycloalcan-2-on-1-carboxylique.

9. Procédé selon la revendication 8, dans lequel le composé ester représenté par la formule générale [2] est un ester d'acide bromoacétique, un ester d'acide malonique, un ester d'acide 2-méthyl-malonique, un ester d'acide 2-fluoromalonique, un ester d'acide 2-méthylacétoacétique ou un ester d'acide cyclohexan-2-on-1-carboxylique.

10. Procédé selon la revendication 2, dans lequel la (3S,4S)-3-[(R)-1-(tert-butyldiméthylsilyloxy)-éthyl]-4-éthoxy-carbonylméthyl-2-azétidinone est préparée à partir du bromoacétate d'éthyle et de la (3S,4R)-3-[(R)-1-(tert-butyldiméthylsilyloxy) -éthyl] -4-phénylthio-2-azétidinone.

11. Procédé selon la revendication 3, dans lequel la (3S,4S)-3 -[(R)-1-(tert-butyldiméthylsilyloxy)-éthyl]-4-di(méthoxy-carbonyl) -méthyl-2-azétidinone est préparée à partir du malonate de diméthyle et de la (3S,4R)-3-[(R)-1-(tert-butyldiméthylsilyloxy)-éthyl]-4-phénylthio-2-azétidinone.

12. Procédé selon la revendication 3, dans lequel la (3S,4S)-3-[(R)-1-(tert-butyldiméthylsilyloxy)-éthyl]-4-[1,1-di(éthoxycarbonyl)éthyl-2-azétidinone est préparée à partir du 2-méthylmalonate de diéthyle et de la (3S,4R)-3-[(R)-1-(tert-butyldiméthylsilyloxy)-éthyl]-4-phénylthio-2-azétidinone.

13. Procédé selon la revendication 3, dans lequel la (3S,4S)-3 -[(R)-1-(tert-butyldiméthylsilyloxy)-éthyl]-4-di(éthoxycarbonyl)fluorométhyl-2-azétidinone est préparée à partir du 2-fluoromalonate de diéthyle et de la (3S,4R)-3-[(R)-1-(tert-butyldiméthylsilyloxy)-éthyl]-4-phénylthio-2-azétidinone.

14. Procédé selon la revendication 3, dans lequel la (3S,4S)-3-[(R)-1-(tert-butyldiméthylsilyloxy)-éthyl]-4-(1-acétyl-1-éthoxycarbonyléthyl)-2-azétidinone est préparée à partir du 2-méthylacétoacétate d'éthyle et de la (3S,4R)-3-[(R)-1-(tert-butyldiméthylsilyloxy)-éthyl]-4-phénylthio-2-azétidinone.

15. Procédé selon la revendication 3, dans lequel la (3S,4S]-3 -[(R]-1-(tert-butyldiméthylsilyloxy) -éthyl]-4-(1-acétyl-1-allyloxycarbonyléthyl)-2-azétidinone est préparée à partir du 2-méthylacétoacétate d'allyle et de la (3S,4R)-3-[(R) -1- (tert-butyldiméthylsilyloxy) -éthyl] -4-phénylthio-2-azétidinone.

16. Procédé selon la revendication 3, dans lequel la (3S, 4S) -3- [(R) -1- (tert-butyldiméthylsilyloxy) -éthyl] -4(1-acétyl-1-benzyloxycarbonyléthyl)-2-azétidinone est préparée à partir du 2-méthylacétoacétate de benzyle et de la (3S,4R)-3-[(R)-1-(tert-butyldiméthylsilyloxy)-éthyl]-4-phénylthio-2-azétidinone.

17. Procédé selon la revendication 3, dans lequel la (3S,4S)-3-[(R)-1-(tert-butyldiméthylsilyloxy)-éthyl]-4-[1,1-di(allyloxycarbonyl)éthyl]-2-azétidinone est préparée à partir du 2-méthylmalonate de diallyle et de la (3R,4S)-3-[(R)-1-(tert-butyldiméthylsilyloxy)-éthyl]-4-phénylthio-2-azétidinone.

18. Procédé selon la revendication 3, dans lequel la (3S,4S)-3-[(R)-1-(tert-butyldiméthylsilyloxy)-éthyl]-4-[(RS)-1-allyloxycarbonyl-cyclohexan-2-on-1-yl] -2-azétidinone est préparée à partir de la 2-allyloxycarbonylcyclohexanone et de la (3R,4S)-3 -[(R)-1-(tert-butyldiméthylsilyloxy)-éthyl]-4-phénylthio-2-azétidinone.

19. Procédé selon l'une quelconque des revendications précédentes, dans lequel le composé du cuivre est un complexe sulfure de diméthyle/bromure cuivreux.
